# EUROPEAN PATENT SPECIFICATION

(11) **EP 3 946 528 B1**
(45) Date of publication and mention of the grant of the patent: **17.06.2026**
(21) Application number: 20783209.8
(22) Date of filing: 27.03.2020
(51) Int. Cl.: A61M 13/00, A61M 16/08, A61M 16/16

(54) **TUBE FOR SURGICAL GAS SUPPLY PRESSURE SENSING**
TUBUS ZUR MESSUNG DES DRUCKS IN DER CHIRURGISCHEN GASVERSORGUNG
TUBE POUR LA DÉTECTION DE PRESSION D'ALIMENTATION EN GAZ CHIRURGICAL

(30) Priority: 29.03.2019 US 201962826208 P
(43) Date of publication of application: 09.02.2022
(73) Proprietor: Fisher & Paykel Healthcare Limited, East Tamaki Auckland 2013 (NZ)
(72) Inventor: TEH, Eu-Lee, Auckland, 2013 (NZ); PEGMAN, Benjamin Elliot Hardinge, Auckland, 2013 (NZ); GELL, Zane Paul, Auckland, 2013 (NZ); BUCKELS, Katie-Ann Jane, Auckland, 2013 (NZ); ARULANDU, Abigail Sharmini Rajen, Auckland, 2013 (NZ); FISCHER, Christian Francis, Auckland, 2013 (NZ); GREENFIELD, James Robert Jarmey, Auckland, 2013 (NZ); SOMERVILLE, Jemma Tamsin, Auckland, 2013 (NZ); WARNER, Zach Jonathan, Auckland, 2013 (NZ)
(74) Representative: Ström & Gulliksson AB
(86) International application number: PCT/IB2020/052893
(87) International publication number: WO 2020/201946

(56) References cited:
- EP-B1- 2 247 335
- WO-A1-2006/019323
- WO-A1-2015/125080
- WO-A1-2015/142192
- WO-A1-2016/154607
- WO-A1-2018/097738
- WO-A1-2018/097738
- WO-A1-2018/106127
- WO-A2-2008/077080
- WO-A2-2012/122263
- CA-A1- 2 840 625
- CN-A- 108 619 601
- US-A- 5 328 458
- US-A1- 2008 105 257
- US-A1- 2014 005 650
- US-A1- 2014 188 038
- US-A1- 2016 001 032
- US-A1- 2016 325 056
- US-A1- 2018 132 895
- US-B2- 9 084 628

## Description

### FIELD OF THE DISCLOSURE

This application claims the benefit under 35 U.S.C. § 119(e) as a nonprovisional application of U.S. Prov. App. No. 62/826,208 filed on March 29, 2019. The present disclosure relates in some embodiments to humidifier systems and components of humidifier systems configured to supply gases to a patient, in particular to monitoring pressure in a surgical cavity of the patient.

### BACKGROUND

Various medical procedures require the provision of gases (such as heated gases) to a patient during the medical procedure. Medical procedures such as closed type medical procedures and open type medical procedures involve delivering gases to the patient, typically carbon dioxide or other similar gases. The gases can be delivered via an interface to the patient, which can include any suitable medical instrument, for example, a cannula, a diffuser, any directed gas flow accessory, and/or others.

In closed type medical procedures, an insufflator is arranged to deliver gases to a body cavity of the patient to inflate the body cavity and/or to resist collapse of the body cavity during the medical procedure. Examples of such medical procedures include laparoscopy and endoscopy, although an insufflator may be used with any other type of medical procedure as required. Endoscopic procedures enable a medical practitioner to visualize a body cavity by inserting an endoscope or the like through natural openings or small puncture(s) to generate an image of the body cavity. In laparoscopy procedures, a medical practitioner typically inserts a surgical instrument through natural openings or small puncture(s) to perform a surgical procedure in the body cavity. In some cases, an initial endoscopic procedure may be carried out to assess the body cavity, and then a subsequent laparoscopy carried out to operate on the body cavity. Endoscopic and/or laparoscopic procedures are widely used, for example, within the peritoneal cavity, or during a thoracoscopy, mediastinoscopy, upper or lower GI procedure (e.g., colonoscopy, gastroscopy, duodenoscopy, jejunoscopy, ileoscopy, or endoscopic retrograde cholangiopancreatography), arthroscopy, cystoscopy or ureteroscopy, bronchoscopy, sinus surgery, or other procedures as being some non-limiting examples. In at least some of these procedures, it can be beneficial for pressure to be maintained within a small cavity.

In open type medical procedures, such as open surgeries, gases are used to fill a surgical cavity, with excess gases spilling outward from the opening. The gases can also be used to provide a layer of gases over exposed internal body parts where there is no discernible cavity. For these procedures, rather than serving to inflate a cavity, the gases can be used to prevent or reduce desiccation and infection by covering exposed internal body parts with a layer of heated, humidified, sterile gases. In open surgeries, pressure may need to be measured, for example, to identify more clearly an occlusion relating to a diffuser or gases pathway, misconnection, and/or misuse of surgical instruments.

The gas pressure sensing systems and/or components thereof disclosed herein can be used, for example, in connection with endoscopic, laparoscopic, or open procedures that can benefit from maintaining pressure within a small cavity, including but not limited to others as disclosed herein.

An apparatus for delivering gases during these medical procedures can include an insufflator arranged to be connected to a remote source of pressurized gases, such as a gases supply system in a hospital. The apparatus can be operative to control the pressure and/or flow of the gases from the gases supply to a level suitable for delivery into the body cavity, usually via a cannula or needle connected to the apparatus and inserted into the body cavity. The apparatus can also be operative to control the pressure and/or flow of the gases via a diffuser arranged to diffuse gases over and into the wound or surgical cavity, such as in an open surgery. In many cases, a humidifier is operatively coupled to the insufflator. A controller of the apparatus can energize a heater of the humidifier located in the gases flow path to humidify the gases stream prior to entering the patient's body cavity. The humidified gases can be delivered to the patient via further tubing, which may also be heated. The insufflator and humidifier can be located in separate housings that are connected together via suitable tubing and/or electrical connections, or located in a common housing arranged to be connected to a remote gas supply via suitable tubing. An example of a prior art arrangement for delivering gases during medical procedures is disclosed in WO2018/097738, relating to a high flow luer connector. Other examples of prior art arrangements for delivering gases during medical procedures are disclosed in US2016/001032, US2008/105257, and WO2016/154607. The document US20161001032 A1, for example, discloses a multi-lumen breathing circuit including a flexible printed circuit board assembly.

### SUMMARY OF THE INVENTION

The present invention provides a tube for delivering a flow of gases in a medical gases delivery system that is configured to supply gases to a patient, as defined in claim 1. Further preferred embodiments of the present invention are defined in the dependent claims.

### SUMMARY OF THE DISCLOSURE

Reference will now be made to various exemplary embodiments of aspects of the present disclosure. Embodiments or aspects disclosed herein which do not fall under the scope of the appended claims do not form part of the present invention, but are useful for understanding the principles of the invention. The scope of the present invention is defined by the appended claims.

For standard surgical gas supplies (such as insufflator units), the operation of gas delivery is usually pulsatile, either partially or fully, due to the need for measuring the pressure at the gas supply unit rather than at the surgical cavity. The insufflator unit pulses gas down a gas delivery line to the patient. The gas flow is paused for the pressure inside the surgical cavity to be read at the insufflator unit to determine the appropriate gas flow for the next pulse. This method of pressure sensing can result in an undesirably reduced surgical cavity due to the delay in measuring pressure from the cavity and then delivering an appropriate response. Further, restrictions in the delivery tube can obstruct the pulses from the insufflator, and hamper the ability to sense pressure at the insufflator due to the restriction. It can also result in instability in the surgical cavity when there are significant leaks, which can negatively interact with the gas control algorithms used to pulse the gas flow. Additionally, when the flow is stopped to allow pressure reading, a backflow of gas is created so that the lens of a viewing instrument, for example, a scope, is more likely to be undesirably in contact with the humid air inside the surgical cavity, thereby causing fogging of and/or condensation formation on the lens which can impede vision.

The present disclosure provides systems and methods for pressure sensing. Various sensors, such as a pressure sensor, a flow rate sensor, a pressure-relief valve, a strain gauge, a force sensor, a temperature sensor, or otherwise, can be positioned in a connector of the cannula or in a tube coupled to the cannula. A pressure tap or pressure line that is connected to a pressure sensor can be included, where the pressure sensor is positioned distal to the cannula/cavity.

The various sensors can determine pressure in the surgical cavity. The measured/determined pressure can be used to control a gases source to provide gases to the surgical cavity. The pressure sensing disclosed herein can advantageously allow for more advanced flow algorithms by removing the need for the gas flow to stop for pressure measurement, and/or optimizing a gas flow stop for measurement (for example, by preventing backflow, decreasing flow stop time, and/or otherwise). This can allow for better pressure and/or flow control, which can help mitigate instability in the surgical cavity or insufficient insufflation, and when combined with other features (such as a directed gas flow cannula), can provide flow profiles that help mitigate problems such as smoke accumulation in the surgical cavity and/or impairment to vision.

The pressure sensing systems and methods disclosed herein can also be combined with other features of the medical gases delivery system, such as reduced restriction at gas connection, a tube set with less friction, a tube set with a consistent diameter, and/or a tube set with multiple connections and/or gas supply sources. A tube set includes multiple components. A tube set optionally includes at least one tube with a heater in the tube, the tube including a connector at each end. One connector can be configured to connect to the insufflator. The other connector can be configured to connect to the surgical cannula. The tube set can optionally include a filter that is downstream of the humidifier to filter gases, for example, air prior to introducing the air into the surgical cavity via the cannula, or prior to introducing the gases into other interfaces, for example, a diffuser, which delivers carbon dioxide.

The reduced restriction of the gas path can help reduce latency in the gas supply system, reduce restriction for gas flow, and may help improve pressure sensing capability. Optimizing the restriction of the gas path can improve operation of the control system. The pressure sensing systems and methods disclosed herein can act as feedback for a control system in the gas supply so that the pneumatic components can be operated to insufflate a patient for surgery. The pressure sensing systems and methods disclosed herein can allow the gas supply to better handle intentional leaking and venting for smoke removal using a venting cannula or venting attachment to a cannula or a venting port. The pressure sensing systems and methods disclosed herein can also be combined with a heated cannula, a directed gas flow cannula and/or accessory, any other interfaces, and/or an optimized humidity source, which can allow for specific flow algorithms that enhance the functionality of these technologies (for example, a continuous flow for the directed gas flow).

The present disclosure provides examples of a medical gases delivery system. The system can comprise a surgical cannula for insertion into a surgical cavity, the cannula comprising: a cannula body including a gases port, wherein the gases port can be configured to be operably coupled to a gases supply; and a cannula shaft coupled to the cannula body, a free end of the cannula shaft configured to be inserted into a surgical cavity for delivering a medical instrument and/or a flow of gases to the surgical cavity; and a sensor configured to measure a characteristic of the flow of gases, the surgical cannula, or the surgical cavity, wherein the sensor can be in electrical communication with a processor and the processor can be configured to determine a pressure inside the surgical cavity based at least in part on the characteristic measured by the sensor. A measured characteristic could include one or more quantitative or qualitative features.

In a configuration, the sensor can comprise a pressure sensor.

In a configuration, the surgical cannula can further comprise a pressure channel in fluid communication with a gases path of the cannula, the sensor located in the pressure channel.

In a configuration, the pressure channel can comprise a medium configured to react to the pressure inside the surgical cavity such that the reaction can be measured by the sensor.

In a configuration, the medium can deform upon exposure to the pressure inside the surgical cavity.

In a configuration, the pressure sensor can comprise a pressure-sensing probe with an elongate body extending from outside the surgical cannula through an orifice of the surgical cannula and a lumen of the cannula shaft.

In a configuration, the pressure sensor can be positioned at a location past seals in the cannula and the pressure sensor is in fluid communication with the lumen of the cannula shaft.

In a configuration, the elongate body of the probe can comprise a wire bendable into a predetermined shape.

In a configuration, the elongate body of the probe can comprise a cannula insert forming an internal cannula inside a lumen of the surgical cannula, the cannula insert comprising one or more seals.

In a configuration, the pressure sensor can be configured to be attached to the medical instrument.

In a configuration, the system can further comprise a pressure port in fluid communication with the surgical cavity and adjacent the surgical cannula, the pressure sensor located in the pressure port.

In a configuration, the system can further comprise a pressure-sensing cannula in fluid communication with the surgical cavity and adjacent the surgical cannula, the pressure sensor located in the pressure-sensing cannula.

In a configuration, the sensor can comprise a strain gauge.

In a configuration, the strain gauge can be embedded in a wall of the surgical cannula.

In a configuration, the strain gauge can be located in the cannula shaft.

In a configuration, the system can further comprise a balloon configured to surround a portion of the cannula shaft and an incision site on a patient's skin, the balloon configured to form an airtight seal with the cannula shaft and the patient's skin, the strain gauge located on a wall of the balloon.

In a configuration, the system can further comprise an inflatable attachment configured to surround a portion of the cannula shaft and an incision site on a patient's skin, when inflated, the inflatable attachment configured to form an airtight seal with the cannula shaft and the patient's skin.

In a configuration, the inflatable attachment can comprise a balloon, the strain gauge located on a wall of the balloon.

In a configuration, the inflatable attachment can comprise one or more retention members, the strain gauge located on or in the one or more retention members, or in a channel in fluid communication with the one or more retention members.

In a configuration, the one or more retention members can comprise at least one member located under the patient's skin.

In a configuration, the one or more retention members can comprise at least one member located above the patient's skin.

In a configuration, the one or more retention members can form a unitary construction.

In a configuration, the strain gauge can comprise an attachment configured to attach to a patient's skin near or adjacent the cannula shaft.

In a configuration, the sensor can comprise a flow sensor.

In a configuration, the flow sensor can be located in the cannula shaft, the flow sensor configured to measure a flow rate of the flow of gases delivered at a continuous flow rate or a flow rate of a known leak orifice in the system.

In a configuration, the continuous flow rate can comprise a cyclic flow that is greater than zero.

In a configuration, the continuous flow rate can be greater than zero. The continuous flow rate may be at a substantially constant level greater than zero.

In a configuration, the continuous flow rate can comprise a constant flow rate.

In a configuration, the surgical cannula further can comprise a pressure channel in fluid communication with a gases path of the cannula, wherein the flow sensor is located upstream of the pressure channel.

In a configuration, the flow sensor can be located in a secondary pressure line connected to the gases port.

In a configuration, they system can further comprise a pressure-sensing cannula in fluid communication with the surgical cavity and adjacent the surgical cannula, the flow sensor coupled to a gases port of the pressure-sensing cannula.

In a configuration, the flow sensor can be located in a venting attachment coupled to the gases port of the pressure-sensing cannula, the venting attachment comprising a known orifice with a leak flow rate.

In a configuration, the sensor can comprise one or more pressure-indicating valves.

In a configuration, a plurality of pressure-indicating valves can be located on a wall of the cannula shaft, the plurality of pressure-indicating valves configured to open at different set pressure values.

In a configuration, a single pressure-indicating valve can be located on a wall of the cannula shaft, the single pressure-indicating valve configured to open at an adjustable set pressure value.

In a configuration, the single pressure-indicating valve can be configured to prevent the pressure inside the surgical cavity from exceeding the adjustable set pressure value.

In a configuration, the processor can be configured to determine a pressure inside the surgical cavity in real time or near real time when the flow of gases to the surgical cavity is not paused.

In a configuration, the processor can be part of a controller of the gases supply. In a configuration, the processor can be part of a controller of a humidifier. In a configuration, the processor can be in the controller of the gases supply and/or the controller of the humidifier. In a configuration, the processor can be embedded within the surgical cannula.

In a configuration, the sensor can be configured to detect over-pressure and/or under-pressure in the system, and/or undesirably high or low flow rates of the flow of gases.

In a configuration, signals from the sensor can be configured to be used to detect undesirable or improper connections, and/or inappropriate connections for a particular surgical application.

The present disclosure provides examples of a pressure-sensing system in a medical gases delivery system. The system can comprise a gases conduit comprising: an elongate body including a lumen extending therethrough; a gases inlet end operably coupled to a gases supply; and a gases outlet end operably coupled to a surgical cannula, wherein the gases inlet end and gases outlet end can be located on opposite ends of the lumen; and a sensor configured to measure a characteristic of the flow of gases, the gases conduit, or the gases supply, wherein the sensor can be in electrical communication with a processor and the processor can be configured to determine a pressure inside the surgical cavity based at least in part on the characteristic measured by the sensor.

In a configuration, the sensor can comprise a pressure sensor.

In a configuration, the pressure sensor can be located at or near the gases inlet end.

In a configuration, the system can comprise a connector coupled to the gases inlet end, wherein the pressure sensor is located at the connector.

In a configuration, the system can comprise a connector coupled to the gases outlet end, wherein the pressure sensor is located at the connector.

In a configuration, the sensor can comprise an expansion ring configured to deform in response to the pressure inside the surgical cavity.

In a configuration, the expansion ring can be located at or near the gases inlet end.

In a configuration, the system can comprise a connector coupled to the gases outlet end, wherein the expansion ring is located at the connector.

In a configuration, the sensor can comprise a heater wire in the elongate body, the heater wire configured to deform in response to the pressure inside the surgical cavity.

In a configuration, the sensor can comprise a flow sensor in fluidic communication with the lumen.

In a configuration, the system can comprise a connector attached to the conduit at the gases inlet end or the gases outlet end, wherein the flow sensor can be coupled to the connector, the connector comprising a known orifice with a known leak flow rate.

In a configuration, the flow sensor can be located in elongate body along the conduit.

In a configuration, the processor can be part of a controller of the gases supply. In a configuration, the processor can be part of a controller of a humidifier. In a configuration, the processor can be in the controller of the gases supply and/or the controller of the humidifier. In a configuration, the processor can be embedded within the gases conduit.

In a configuration, the sensor can be configured to detect over-pressure and/or under-pressure in the system, and/or undesirably high or low flow rates of the flow of gases.

In a configuration, signals from the sensor can be configured to be used to detect undesirable or improper connections, and/or inappropriate connections for a particular surgical application.

The present disclosure provides examples of a tube or tube-set for delivering a flow of gases in a medical gases delivery system. The tube or tube-set can comprise at least one tube including a first end and a second end; the tube defining a lumen to transport gases through it; a first connector at the first end and a second connector at the second end of the tube; and a sensor configured to measure a characteristic of the flow of gases or a characteristic of a component of the tube-set, wherein the sensor can be in electrical communication with a processor and the processor is configured to determine a pressure in the tube-set based at least in part on the characteristic measured by the sensor, and wherein the sensor can comprise a pressure sensor.

According to the invention, the present disclosure provides examples of a tube or tube-set for delivering a flow of gases in a medical gases delivery system that is configured to supply gases to a patient. The tube comprises a first end and a second end; a first connector at the first end and a second connector at the second end of the tube; an inner conduit defining a lumen to transport gasses therethrough; an outer conduit, coaxial with and surrounding the inner conduit such that the inner and outer conduits define a dual wall tube defining a secondary lumen between walls of the inner and outer conduits; and a pressure sensor arranged in the secondary lumen to measure a pressure in the secondary lumen, wherein the pressure sensor is configured to be in electrical communication with a processor; and wherein the secondary lumen, is not configured for delivery or removal of gases therethrough.

In a configuration, the at least one tube can comprise a first conduit and a second conduit, the first and second conduits being co-axial with each other.

In a configuration, the first conduit can be positioned within the second conduit and being surrounded by the second conduit, the first conduit and second conduit defining a dual wall tube.

In a configuration, the first connector and/or the second connector can comprise a Luer connector.

In a configuration, the first connector and/or the second connector can comprise a Luer connector, wherein the Luer connector comprises a resilient outer cover.

In a configuration, the pressure sensor can be located at the first connector.

In a configuration, the pressure sensor can be located at the second connector.

In a configuration, the sensor can comprise an expansion ring configured to deform in response to the pressure.

In a configuration, the expansion ring can be located at the first connector.

In a configuration, the expansion ring can be located at the second connector.

In a configuration, the sensor can comprise a heater wire in the tube, the heater wire configured to deform in response to the pressure.

In a configuration, the sensor can comprise a flow sensor in fluidic communication with a lumen of the tube.

In a configuration, the flow rate sensor can be coupled to the first or second connector, the first or second connector comprising a known orifice with a leak flow rate that is determined by the flow rate sensor.

In a configuration, the flow sensor can be located in a wall of the tube.

In a configuration, the processor can be part of a controller of the gases supply. In a configuration, the processor can be part of a controller of a humidifier. In a configuration, the processor can be in the controller of the gases supply and/or the controller of the humidifier. In a configuration, the processor can be embedded within the at least one tube.

In a configuration, the tube or tube-set can comprise a filter that is located downstream of a humidifier.

In a configuration, the at least one tube can comprise a delivery tube that connects a gases supply to a humidifier and a supply tube that connects the humidifier to a cannula.

In a configuration, the at least one tube can comprise a delivery tube that connects a gases supply to a humidifier and a supply tube that connects the humidifier to a medical instrument.

In a configuration, the medical instrument can comprise a diffuser.

In a configuration, the medical instrument can comprise a directed gas flow accessory.

The present disclosure provides examples of a surgical humidification system comprising the tube or tube-set of any of the configurations described above, and a humidifier comprising a humidification chamber, the first or second connector coupled to an outlet of the humidification chamber.

In a configuration, the humidifier can be configured to humidify the flow of gases prior to introducing the flow of gases into a surgical cavity.

In a configuration, the system can comprise an insufflator, wherein the insufflator is the gases supply.

In a configuration, the sensor can be configured to detect over-pressure and/or under-pressure in the system, and/or undesirably high or low flow rates of the flow of gases.

In a configuration, signals from the sensor can be configured to be used to detect undesirable or improper connections, and/or inappropriate connections for a particular surgical application.

The present disclosure provides examples of a medical gases delivery system. The system can comprise a medical instrument for insertion into a surgical cavity, the medical instrument being coupled to a gases supply and in fluid communication with the surgical cavity; and a sensor configured to measure a characteristic of the flow of gases, the medical instrument, or the surgical cavity, wherein the sensor can be in electrical communication with a processor and the processor is configured to determine a pressure inside the surgical cavity based at least in part on the characteristic measured by the sensor.

In a configuration, the sensor can comprise a pressure sensor.

In a configuration, the sensor can comprise a flow sensor.

In a configuration, the flow sensor can be configured to measure a flow rate of the flow of gases delivered at a continuous flow rate or a flow rate of a known leak orifice in the system.

In a configuration, the continuous flow rate can comprise a cyclic flow that is greater than zero.

In a configuration, the continuous flow rate can be constantly greater than zero.

In a configuration, the continuous flow rate can comprise a constant flow rate.

In a configuration, the sensor can be configured to be attached to an end connector coupled to the medical instrument.

In a configuration, the medical instrument can comprise a diffuser.

In a configuration, the medical instrument can comprise a directed gas flow accessory.

In a configuration, the processor can be configured to determine a pressure inside the surgical cavity in real time or near real time when the flow of gases to the surgical cavity is not paused.

In a configuration, the processor can be part of a controller of the gases supply. In a configuration, the processor can be part of a controller of a humidifier. In a configuration, the processor can be in the controller of the gases supply and the controller of the humidifier.

In a configuration, the sensor can be configured to detect over-pressure and/or under-pressure in the system, and/or undesirably high or low flow rates of the flow of gases.

In a configuration, signals from the sensor can be configured to be used to detect undesirable or improper connections, and/or inappropriate connections for a particular surgical application.

The present disclosure provides examples of a method of sensing pressure within a medical gases delivery system comprising: inserting a surgical cannula into a surgical cavity, the cannula comprising a cannula body including a gases port, wherein the gases port is operably coupled to a gases supply; and a cannula shaft coupled to the cannula body, wherein a free end of the cannula shaft can be inserted into the surgical cavity; flowing gases into the surgical cavity; sensing a characteristic of the flow of gases, a component of a tube-set, the surgical cannula, or the surgical cavity; transmitting data relating to the characteristic of the flow of gases, a component of the tube-set, the surgical cannula, or the surgical cavity to a processor; and estimating a pressure inside the surgical cavity via the processor based at least in part on the characteristic measured by the sensor, wherein estimating the pressure can occur in real-time or near-real time without pausing the flowing of gases into the surgical cavity.

In a configuration, the method can comprise outputting the pressure estimated by the processor to a display.

In a configuration, the processor can be part of a controller of the gases supply. In a configuration, the processor can be part of a controller of a humidifier. In a configuration, the processor can be in the controller of the gases supply and/or the controller of the humidifier. In a configuration, the processor can be embedded within the surgical cannula.

The present disclosure provides examples of a method of sensing pressure within a medical gases delivery system comprising: inserting a medical instrument into a surgical cavity, the medical instrument being coupled to a gases supply and in fluid communication with the surgical cavity; delivering gases into the surgical cavity; sensing a characteristic of the flow of gases, a component of a tube-set, the medical instrument, or the surgical cavity; transmitting data relating to the characteristic of the flow of gases, a component of the tube-set, the medical instrument, or the surgical cavity to a processor; and estimating a pressure inside the surgical cavity via the processor based at least in part on the characteristic measured by the sensor, wherein estimating the pressure can occur in real-time or near-real time without pausing the delivering of gases into the surgical cavity.

In a configuration, the method can comprise outputting the pressure estimated by the processor to a display.

In a configuration, the processor can be part of a controller of the gases supply. In a configuration, the processor can be part of a controller of a humidifier. In a configuration, the processor can be in the controller of the gases supply and the controller of the humidifier.

In a configuration, the medical instrument can comprise a diffuser.

In a configuration, the medical instrument can comprise a directed gas flow accessory.

The present disclosure provides examples of a non-transitory computer-readable medium having stored thereon computer executable instructions that, when executed on a processing device, cause the processing device to perform any method according to any one of the configurations described above.

### BRIEF DESCRIPTION OF THE DRAWINGS

These and other features, aspects, and advantages of the present disclosure are described with reference to the drawings of certain embodiments, which are intended to schematically illustrate certain embodiments and not to limit the disclosure.
Figure 1A illustrates schematically an example medical gases delivery system in use in surgery.
Figure 1B illustrates schematically a block diagram of an example gas flow control system of a medical gases delivery system.
Figures 2A-2C illustrate schematically examples of a gases delivery system and an instrument stack.
Figure 2D illustrates schematically a gases delivery system with a pressure sensing feature coupled to an example diffuser.
Figure 2E illustrates schematically a gases delivery system with a pressure sensing feature coupled to an example directed gas flow accessory.
Figure 3 illustrates a cross-sectional view of a secondary pressure line, such as a tube according to some embodiments.
Figure 4A illustrates schematically a cross-sectional view of an example expansion ring for pressure sensing.
Figures 4B-4C illustrate perspective and cross-sectional views of an example expansion ring.
Figure 4D illustrates schematically a cross-sectional view of an example expansion ring in a connector to a gas port of a cannula.
Figures 5A-5C illustrate schematically cross-sectional views of examples of a controlled leak structure for pressure sensing.
Figure 6 illustrates schematically an example pressure tap near an outlet of the gases supply.
Figure 7A illustrates schematically a cross-sectional view of an example gases delivery conduit with a helical heater wire.
Figure 7B illustrates schematically example deformation of a heater wire.
Figure 8A illustrates schematically a cross-sectional view of an example gases delivery tube with an in-line flow sensor.
Figure 8B illustrates schematically a cross-sectional view of an example modular flow sensor coupled to a gases delivery tube.
Figure 9 illustrates schematically an example pressure tap near a gases port of the cannula.
Figure 10 illustrates schematically a cross-sectional view of an example cannula with a pressure channel.
Figures 11A-11C illustrate schematically cross-sectional views of example cannulas with a pressure channel containing a medium.
Figure 12 illustrates schematically a cross-sectional view of an example cannula with a strain gauge.
Figure 13 illustrates schematically a cross-sectional view of an example cannula with a flow rate sensor.
Figure 14 illustrates schematically a cross-sectional view of an example cannula with a pressure channel.
Figure 15 illustrates schematically a cross-sectional view of an example strain balloon over a surgical incision site inserted with a cannula.
Figure 15A illustrates schematically a cross-sectional view of a cannula and an example retention member inserted under a surgical incision site.
Figure 15B illustrates schematically a cross-sectional view of a cannula and another example retention unit including a first member inserted under a surgical incision site and a second member inserted outside the surgical site.
Figures 16A and 16B illustrate schematically cross-sectional views of example pressure-indicating valves on a cannula.
Figures 16C-16E illustrate schematically cross-sectional views of a cannula incorporating an example pressure-indicating slider.
Figure 17 illustrates schematically a cross-sectional view of a cannula and an example strain attachment.
Figure 18A illustrates schematically a cross-sectional view of a cannula and an example pressure-sensing probe.
Figures 18B-18C illustrate schematically a cross-sectional view and partially exploded view of an example cannula and an example pressure-sensing probe.
Figure 19 illustrates schematically a cross-sectional view of a venting cannula with an example pressure-sensing attachment.
Figure 20 illustrates schematically a cross-sectional view of a gases delivery cannula and an example pressure-sensing cannula.
Figure 21 illustrates schematically a cross-sectional view of a cannula inserted with a medical instrument and a pressure sensor attached to the medical instrument.
Figure 22 illustrates a cross-sectional view of a cannula inserted into a surgical site and an example separate pressure port.

### DETAILED DESCRIPTION

Although certain embodiments and examples are described below, those of skill in the art will appreciate that the disclosure extends beyond the specifically disclosed embodiments and/or uses and obvious modifications and equivalents thereof. Thus, it is intended that the scope of the disclosure herein disclosed should not be limited by any particular embodiments described below.

### Example Medical Gases Delivery Systems

Gases can be introduced to a surgical cavity, such as the peritoneal cavity via an interface, for example, a cannula, inserted through an incision made in patient's body (such as the abdominal wall). The interface, for example, the cannula, can be coupled to an insufflator. The gases flow from the insufflator can be increased to inflate the surgical cavity (such as for example to maintain a pneumoperitoneum, which is a cavity filled with gas within the abdomen). The introduced gases can inflate the surgical cavity. A medical instrument can be inserted through the cannula into the inflated surgical cavity. For example, an endoscope, another scope, or camera unit can be inserted into the cavity and visibility in the cavity can be assisted by insertion of gases, which can be air or carbon dioxide or any other suitable gases. After initial insufflation and insertion of the instrument (such as a laparoscope) through the primary cannula, additional cannulas can be placed in the surgical cavity under laparoscopic observation. Gases and/or surgical smoke can be vented from the surgical cavity using a venting attachment on one of the cannulas placed in the surgical cavity. Smoke can be vented using a passive smoke venting arrangement that is configured to vent smoke due to a pressure differential between the surgical cavity and atmosphere. The passive smoke venting arrangement may include a filter to filter smoke and/or other particulate matter prior to venting the smoke. Alternatively, an active smoke evacuation system may be used to vent or remove smoke from the cavity. For example, the active smoke evacuation system may include a pump or other device that creates suction or a pressure differential to draw smoke out of the surgical cavity. An active smoke evacuation system may include a filter to filter smoke and/or particulate matter prior to venting the smoke. The evacuated gases may be recirculated and delivered back into the surgical cavity.

At the end of the operating procedure, all instruments and cannulas are removed from the surgical cavity, the gases are expelled, and each incision is closed. For thoracoscopy, colonoscopy, sigmoidoscopy, gastroscopy, bronchoscopy, and/or others, some non-limiting examples of which are disclosed elsewhere herein, the same or substantially similar procedure for introducing gases to a surgical cavity can be followed. The quantity and flow of gases can be controlled by the clinician performing the examination and/or automatically by the insufflation system, which can include an insufflator and other components, for example but not limited to a pressure relief valve or otherwise. The insufflator may deliver intermittent or continuous flow. The insufflator can control flow to ensure that the pressure in the surgical cavity is maintained at or around a predetermined range. The pressure allows for the surgical cavity to be inflated to a predetermined volume.

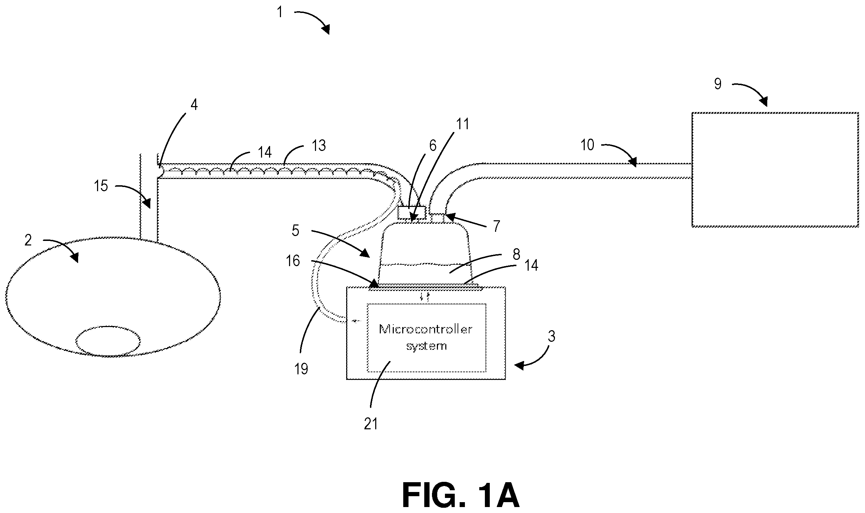

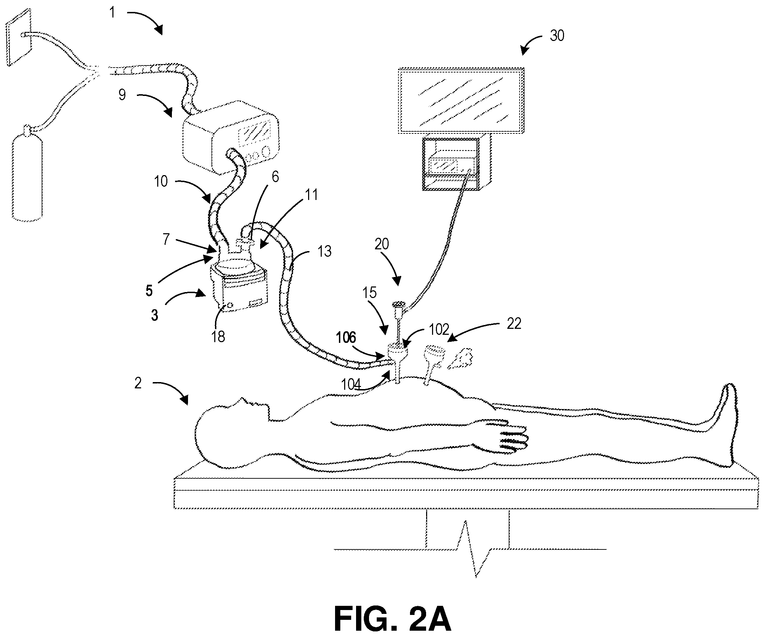

Figures 1A and 2A-C illustrate schematically using an example insufflation system 1 during a medical procedure. Features of Figures 1A and 2A-C can be incorporated into each other. The same features have the same reference numerals in Figures 1A and 2A-C. As shown in Figure 1A, the patient 2 can have an interface, for example, a cannula 15, inserted within a cavity of the patient 2 (for example, an abdomen of the patient 2 in the case of a laparoscopic surgery), as previously described.

As shown in Figures 1A and 2A-C, the cannula 15 can be connected to a gases delivery conduit 13 (for example, via a Luer lock connector 4). The cannula 15 can be used to deliver gases into a surgical site, such as within the cavity of the patient 2 (for example, into the peritoneal cavity to establish a pneumoperitoneum). The cannula 15 can include one or more passages to introduce gases and/or one or more surgical instruments 20 into the surgical cavity. The surgical instrument can be a scope, electrocautery tool, or any other instrument. The surgical instrument 20 can be coupled to an imaging device 30, which can have a screen in the case of the instrument being a scope. Other surgical instruments can also be coupled to different surgical systems. The imaging device 30 can be part of a surgical stack, which can include a plurality of surgical tools and/or apparatuses. In some configurations, the cannula 15 can be used in a system that includes a supplementary gases supply. The supplementary gases supply may be in addition to the insufflator. Multiple cannulae may be used during a surgery and one cannula may be coupled to a supplementary gases supply to deliver continuous flow into the surgical cavity through the cannula 15.

The cannula disclosed herein may optionally include one or more structures that retain the scope in a substantially concentric and/or coaxial orientation relative to the cannula in order to improve visibility through the scope. Having gas pressure sensing external to the gases source can improve delivery of the gases flow and the performance of the concentric and/or coaxial features, for example, to allow a more consistent flow of gases to be delivered to the patient. The gas pressure sensing external to the gases source can allow the pressure measurement to be closer to the patient, which can improve patient safety as the pressure measurement can be more accurate and therefore allowing better control of the delivery of gases to the patient.

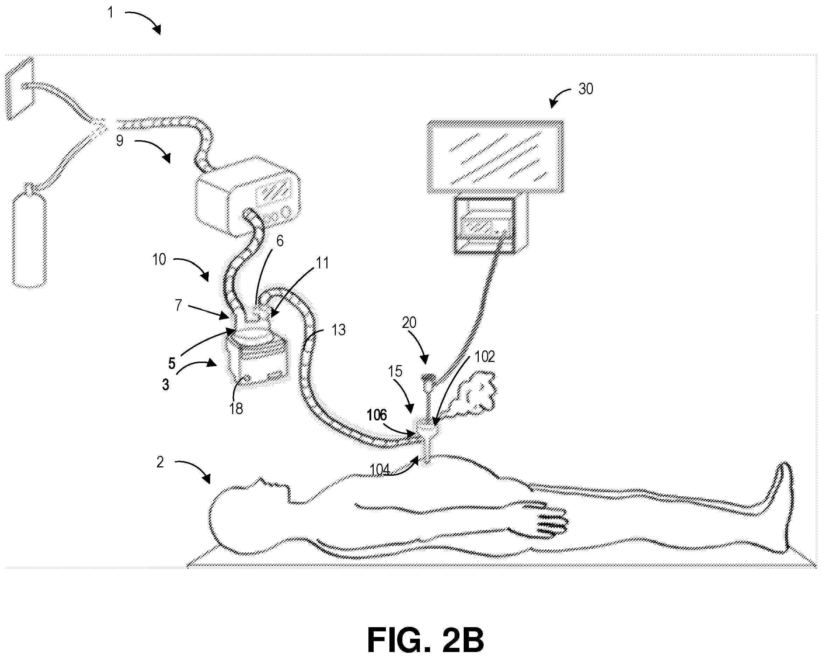

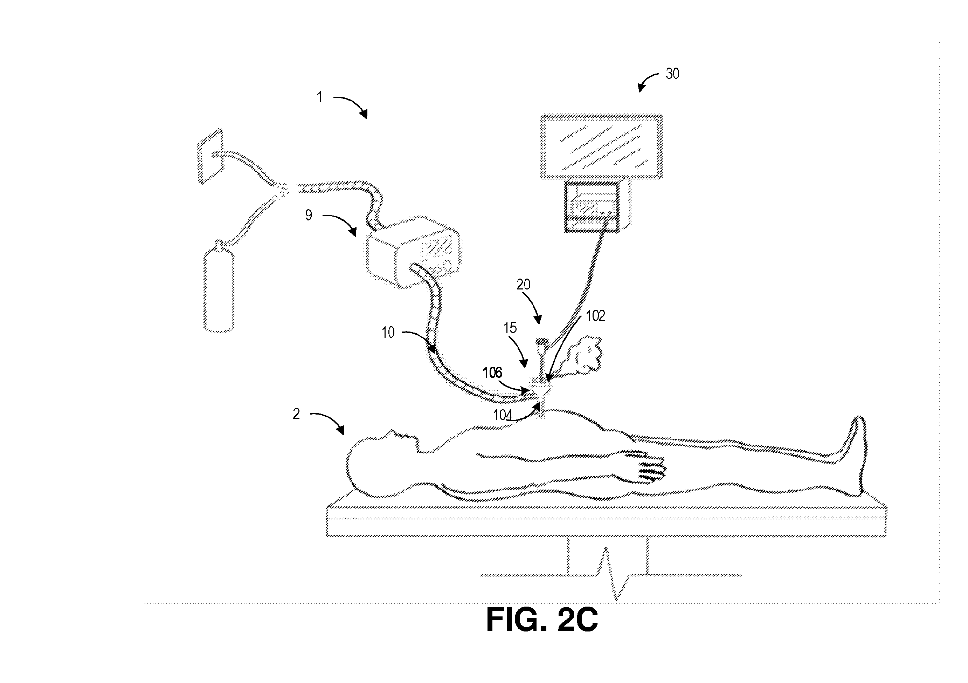

As shown in Figure 2A, the system can include a venting cannula 22, which can have substantially the same features as the cannula 15. The venting cannula may include a leak device coupled to the venting cannula. The leak device may include a valve that allows and/or controls venting. Alternatively, the leak device may have passive and/or active venting structures. The leak device may also be shaped or may include features that control the venting rate out of the surgical cavity. The valve can be automatically controlled by a controller associated with the gases supply (for example, insufflator) and/or by a controller in the humidifier. The valve can also be manually actuated (for example, by turning a tap by hand or by a foot pedal, or otherwise). The leak device can include a filtration system to filter out smoke and the like. The venting cannula 22 can also alternatively be coupled to a recirculation system that is configured to recirculate the gases from the surgical cavity back to the insufflator for re-delivery into the surgical cavity. The gases can also be filtered and/or dehumidified prior to being returned to the insufflator. As shown in Figures 2B and 2C, the cannula 15 can include a venting attachment so that a venting cannula 22 may not be necessary. The cannula 15 may include two or more passages. One passage can be configured to deliver gases and/or the medical instrument into the surgical cavity. Another passage can be configured to vent gases out of the surgical cavity. The venting attachment can include a retrofit attachment, a separate cannula, or can be integrated into the cannula to make the cannula a venting cannula. For example, the venting attachment can form an outlet from the cavity. Venting can increase the gas flow, thereby improving visibility by reducing fog/condensation on the lens of a viewing instrument, for example, lens of a laparoscope, and/or within the cavity.

As shown in Figures 1A, 2A, and 2B, the gases delivery conduit 13 can be made of a flexible plastic and can be connected to a humidifier chamber 5. The humidifier chamber 5 can optionally or preferably be in serial connection to a gases supply 9 via a further conduit 10. The gases supply 9 can be an insufflator, bottled gases, or a wall gases supply. The gases supply 9 can provide the gases without humidification and/or heating. Optionally, the humidifier chamber 5 can be incorporated as part of the gases supply 9. As shown in Figure 1A, an end connector 4 can be connected downstream of the humidifier's outlet 11. A filter 6 can be connected downstream of the humidifier's outlet 11. The filter can also be located along the further conduit 10, or at an inlet of the cannula 15. The filter can be configured to filter out pathogens and particulate matter in order to reduce infection or contamination of the surgical site from the humidifier or gases supply. The gases supply can provide a continuous or intermittent flow of gases. The further conduit 10 can also preferably be made of flexible plastic tubing.

The gases supply 9 can provide one or more insufflation gases, such as, for example, carbon dioxide, to the humidifier chamber 5. The gases can be humidified as they are passed through the humidifier chamber 5, which can contain a volume of water or other liquid 8. The gases are humidified by a passover humidification mechanism in the illustrated humidifier 5.

A humidifier that incorporates the humidifier chamber 5 can be any type of humidifier. The humidifier chamber 5 can include a plastic formed chamber having a metal or otherwise conductive base 14 sealed thereto. The base can be in contact with the heater plate 16 during use. The volume of water 8 contained in the chamber 5 can be heated by a heater plate 16, which can be under the control of a controller 21 of the humidifier. The volume of water 8 within the chamber 5 can be heated such that it evaporates, mixing water vapor with the gases flowing through the chamber 5 to heat and humidify the gases.

The controller 21 can be housed in a humidifier base unit 3, which can also house the heater plate 16. The heater plate 16 can have an electric heating element therein or in thermal contact therewith. One or more insulation layers can be located between the heater plate 16 and the heater element. The heater element can be a base element (or a former) with a wire wound around the base element. The wire can be a nichrome wire (or a nickel-chrome wire). The heater element can also include a multi-layer substrate with heating tracks electrodeposited thereon or etched therein. The controller 21 can include electronic circuitry, which can include a microprocessor for controlling the supply of energy to the heating element. The humidifier base unit 3 and/or the heater plate 16 can be removably engageable with the humidifier chamber 5. The humidifier chamber 5 can also alternatively or additionally include an integral heater.

The heater plate 16 can include a temperature sensor, such as a temperature transducer, a thermistor, or otherwise, which can be in electrical connection with a processor or signal processor. The processor can be part of the controller 21. The processor may be in the humidifier and/or in the gases supply. Alternatively, the processor may be incorporated into a tube, such as the gases delivery conduit, or a portion of the tube. As another alternative configuration, the processor may be a remote processor that is arranged in wireless communication with any sensors or sensing apparatus. The processor includes a processing unit and a data storage unit, for example, a memory unit. The data storage can store measured values for further processing. The heater plate temperature sensor can be located within the humidifier base unit 3. The processor can monitor the temperature of the heater plate 16, which can approximate a temperature of the water 8.

A temperature sensor can also be located at the or near the outlet 11 to monitor a temperature of the humidified gases leaving the humidifier chamber 5 from the outlet 11. The temperature sensor can also be connected to the processor (for example, with a cable or wirelessly). Additional sensors can also optionally be incorporated, for example, for sensing characteristics of the gases (such as temperature, humidity, flow, or others) at a patient end of the gases delivery conduit 13. The humidifier operation can also be controlled based on the temperature of the heater plate and/or the temperature sensor at the chamber outlet 11.

The gases can exit out through the humidifier's outlet 11 and into the gases delivery conduit 13. The gases can move through the gases delivery conduit 13 into the surgical cavity of the patient 2 via the cannula 15, thereby inflating and maintaining the pressure within the cavity. In some cases, the gases leaving the outlet 11 of the humidifier chamber 5 can have a relative humidity of around 100%. As the gases travel along the gases delivery conduit 13, "rain out," that is, condensation when the temperature of the gases decreases below the dew point of the gases, can occur so that water vapor can condense on a wall of the gases delivery conduit 13. Rain out can have undesirable effects, such as detrimentally reducing the water content of the gases delivered to the patient. In order to reduce and/or minimize the occurrence of condensation within the gases delivery conduit 13, a heater wire 14 can be provided within, throughout, or around the gases delivery conduit 13. The heater wire 14 can be electronically connected to the humidifier base unit 3, for example by an electrical cable 19 to power the heater wire. In some embodiments, other heating elements could be included in addition or alternatively, e.g., a conductive ink, or a flexible PCB.

The heater wire 14 can include an insulated copper alloy resistance wire, other types of resistance wire, or other heater element, and/or be made of any other appropriate material. The heater wire can be a straight wire or a helically wound element. An electrical circuit including the heater wire 14 can be located within walls of the gases delivery tube 13. The gases delivery tube 13 can be a spiral wound tube. The heater wire 14 can be spirally wound around an insulating core of the gases delivery conduit 13. The insulating coating around the heater wire 14 can include a thermoplastics material which, when heated to a predetermined temperature, can enter a state in which its shape can be altered and the new shape can be substantially elastically retained upon cooling. The heater wire 14 can be wound in a single or double helix. As alternatives to a heater wire 14, the gases delivery conduit 13 can include any other types of heating element. Measurements by the temperature sensor and/or the additional sensor(s) at the patient end of the conduit 13 can provide feedback to the processor of the controller 21 so that the controller 21 can optionally energize the heater wire to increase and/or maintain the temperature of the gases within the gases delivery conduit 13 so that the gases delivered to the patient can be at or close to a desired temperature. In one example, the outlet set point can be about 37° C. Alternatively, the gases are delivered at a temperature to maintain a core temperature of the patient at a desired level, for example, at a 37°C core temperature. The gases may be heated in the gases delivery conduit 13. The gases may also be heated within the cannula by appropriate heating structures in the cannula. Alternatively or additionally, the system can include additional sensors configured to measure one or more parameters, for example, ambient temperature and ambient humidity sensors; and/or flow sensors, and/or pressure sensors configured to determine flow rate or pressure of flow or determine the pressure within a cavity or in the tube. Additionally or alternatively, the system may also include additional sensors. The sensors can be located upstream, downstream, and/or within the humidifier. The sensors may be configured to determine a parameter of the insufflation gases or one or more parameters of the patient/surgical cavity. Each of the sensors can provide feedback information for controlling the gases supply. For all the sensors disclosed herein, the sensors may be able to communicate wirelessly with the processor or may have a wired connection with the processor.

The controller can, for example, include the microprocessor or logic circuit with associated memory or storage means, which can hold a software program. When executed by the controller 21, the software can control the operation of the insufflation system 1 in accordance with instructions set in the software and/or in response to external inputs. For example, the processor of the controller 21 can be provided with input from the heater plate 16 so that the controller 21 can be provided with information on the temperature and/or power usage of the heater plate 16. The processor of the controller 21 can be provided with inputs of temperature of the gases flow. For example, the temperature sensor can provide input to indicate the temperature of the humidified gases flow as the gases leave the outlet 11 of the humidifier chamber 5. The temperature sensor can be at or near the interface to assist with device performance and/or patient safety monitoring. A flow sensor can also be provided in the same position as or near the temperature sensor or at another appropriate location within the insufflation system 1. The controller 21 can control a flow regulator which regulates the flow rate of gases through the system 1. The regulator can include a flow inducer and/or inhibiter such as a motorized fan. Valves and/or vents can, additionally or alternatively, be used to control the gases flow rate.

A user interface 18 configured to receive input information can be located on the humidifier base unit 3. The user interface 18 can allow a user (such as a surgeon or nurse) to set a desired gases temperature and/or gases humidity level to be delivered. Other functions can also optionally be controlled by the user interface 18, such as control of the heating delivered by the heater wire 14. The controller 21 can control the system 1, and in particular control the flow rate, temperature, and/or humidity of gas delivered to the patient, to be appropriate for the type of medical procedure for which the system 1 is being used. The humidifier base unit 3 can also include a display for displaying to the user the characteristics of the gas flow being delivered to the patient 2.

When in use, the humidifiers described above can be located outside an "operating sterile zone" and/or adjacent the insufflator. As a result, the medical personnel would not be required to touch the humidifier when moving the cannula during the operation to maneuver the medical instruments within the surgical cavity. The humidifier and the humidification fluid therein may not need to be sterilized to the same extent as the medical instruments. Furthermore, the humidifier being located outside the "operating sterile zone" can reduce obstructions to the medical personnel during the operating procedure that may restrict movements of the medical personnel and/or the medical instruments in the already crowded space.

As shown in Figure 2C, the system may be used without a humidifier so that the gases supply 9 can be coupled directly to the cannula 15. The humidifier can be an optional unit. Humidifying the insufflation gases reduces cellular damage or desiccation of tissues in or around the surgical cavity. In an alternative arrangement, the humidifier may be integrated with the insufflator, such that the gases supply includes both gases control elements and humidification elements.

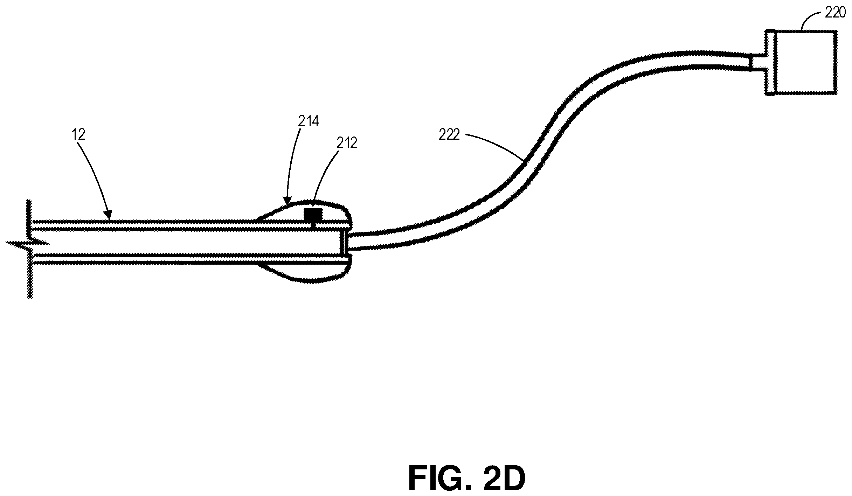

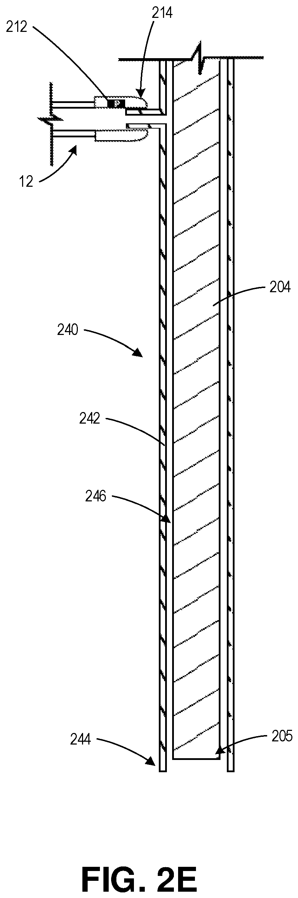

As alternative embodiments relating to the cannula 15, the gases delivery conduit 13 shown in Figures 1A, 2A and 2B and the further conduit 10 shown in Figure 2C, which are examples of a conduit connected directly or indirectly to the gases source, can also be connected to other interfaces. Additional non-limiting examples of an interface are illustrated in Figures 2D and 2E.

As shown in Figure 2D, a conduit 12 is connected directly or indirectly to the gases source and can be coupled to a diffuser 220 via a diffuser tubing 222. The diffuser can be made from a material that is porous and/or non-porous. A gases delivery system including the diffuser 220 can be used, for example, in an open surgery as described above. A pressure sensing feature 212, which can include any of the example pressure sensors or pressure sensing systems disclosed herein, can be located at a distal end of the conduit 12 (for example, in an end connector 214). Alternatively, the pressure sensing system can be located elsewhere in the gases delivery system, including but not limited to the examples disclosed herein, and/or other accessories connected to the conduit 12.

The pressure sensing feature 212 can provide redundancy in the prevention of over-pressure in the gases delivery system that includes an existing safety feature against undesired elevated pressure. The pressure sensing feature 212 can also provide protection against under-pressure, and/or undesirably high or low flow rates. Pressure measurements from the pressure sensing feature 212 can be communicated to the controller 21. For example, if a tube in the system gets occluded (partially or entirely) and the pressure in the system exceeds a predetermined threshold, the pressure sensing feature 12 can communicate with the controller 21, which can in turn limit or disable the supply of gases until the pressure returns to normal levels, that is, below the threshold. Additionally, the pressure sensing feature 212 can detect pressure signals configured to be used in inferring unwanted use cases, which can be related to, for example, unwanted or undesired use of the diffuser 220. The diffuser 220 can cause splatter, bubbling, and/or other unwanted or undesirable effects, which can interfere with proper delivery of the gases. The pressure sensing feature 212 that is located closer to the patient can provide signals used for monitoring characteristics in the pressure signal (such as oscillations), which may be related to the unwanted or undesirable events, such as undesirable connections, improper connections, and/or inappropriate connections for a particular surgical application. The pressure sensing feature 12 can communicate with the controller 21, which can in turn limit the supply of gases, for example, by limiting the pressure and/or flow rate, accordingly.

As shown in Figure 2E, a conduit 12 connected directly or indirectly to the gases source can be coupled to an accessory 240, a cannula and/or medical instrument (for example, a directed gas flow accessory). As illustrated in Figure 2E, the accessory 240 can be a sheath that directly goes over a medical instrument 204, for example, a laparoscope, to deliver insufflation gases to the lens. The cannula and/or medical instrument accessory 240 can be used for localizing insufflation at the lens and/or venting of gases with respect to a surgical cavity of a patient (such as the pneumoperitoneum), allowing insertion of the medical instrument 204 into the surgical cavity through the cannula, or venting of gases near an operating end 205 of the medical instrument 204. The accessory 240 can include a body 242 mountable over at least a portion of a shaft of the medical instrument 204. The body 242 can include an inner lumen. A distal end 244 of the body 242 can include an opening and is arranged in use at or adjacent the operating end 205 of the medical instrument 204. An outer wall of the medical instrument shaft and an inner surface of the accessory 240 can define a gas flow path 246. Gases can be released or introduced into the gas flow path 246 and can exit at the distal end 244 of the accessory 240 and adj acent the operating end 205 of the medical instrument 204. The distal end 244 can be configured to direct gases across the lens of a viewing instrument, for example, a laparoscope.

As shown in Figure 2E, a pressure sensing feature 212, which can include any of the example pressure sensors or pressure sensing systems disclosed herein, can be located at an end of the conduit 12 (for example, in a connector 214). The pressure sensing feature 212 can enable more accurate control over the delivery of gases through the accessory 240 and across the lens to reduce fog/condensation.

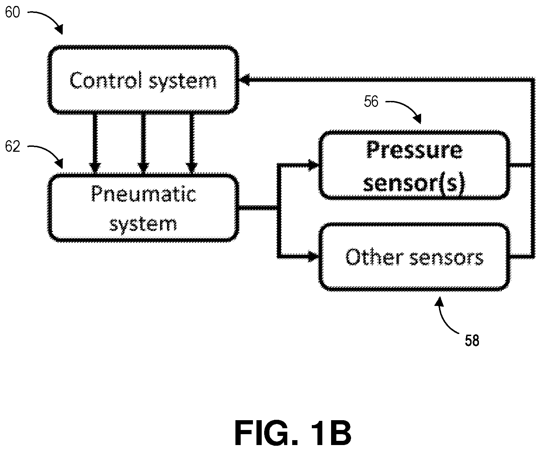

As described above, the present disclosure provides systems and methods for gas supply control of the systems described above. As shown in Figure 1B, the one or more sensor or a sensing apparatus 56, 58 can be incorporated into either a tube set, the cannula, or another component of the system and be used to determine the pressure in the surgical cavity. The one or more sensor or a sensing apparatus 56, 58 can provide feedback to a control system 60, which in turn can control a pneumatic system 62. As noted above, a tube set includes multiple components. A tube set optionally includes at least one tube with a heater in the tube, the tube including a connector at each end. One connector can be configured to connect to the insufflator. The other connector can be configured to connect to the surgical cannula. The tube set can optionally include a filter that is downstream of the humidifier to filter air prior to introducing the air into the surgical cavity via the cannula. The pressure in the surgical cavity as determined by the sensor(s) or sensing apparatus 56, 58 can be used to control the gases flow to the surgical cavity and/or as a safety measure during surgery to prevent over-pressurization and/or damage to the surgical cavity. The pressure measurements can also be used to improve operation of the gases supply and/or humidifier.

The gas flow control system and related sensor configurations disclosed herein can be implemented in any of the medical gases delivery systems disclosed herein, or on a secondary gas source used in combination with any of the medical gases delivery systems.

Examples of pressure sensing devices and methods are described below. The method of attaching the pressure sensing devices to the medical gases delivery system incorporates all necessary electrical connections. All electrical connections can be wired connections or wireless connections. The pressure sensing devices and methods can be incorporated in any of the medical gases delivery systems disclosed herein and/or components thereof, for example, with any gases supply (such as insufflators), gases delivery cannula, or gases delivery conduit. The pressure sensing devices and methods can also be used for any laparoscopic surgery in which pressure inside the surgical cavity needs to be monitored. The pressure sensing devices and methods can provide protection against over-pressure, under-pressure, and/or undesirably high or low flow rates. The pressure sensing device that is located closer to the patient can provide signals used for monitoring characteristics in the pressure signal (such as oscillations), which may be related to the unwanted or undesirable events, such as undesirable connections, improper connections, and/or inappropriate connections for a particular surgical application.

One or more pressure sensor or a pressure sensing apparatus or pressure sensing device disclosed herein can be incorporated into a component of a tube. For example, the pressure sensor or pressure sensing apparatus may be incorporated into the tube body or into a connector of the tube. The pressure sensor or a pressure sensing apparatus can be configured to measure pressure in the tube, which can be used to determine the pressure in the surgical cavity. In one example implementation, the processor equates the measured pressure in the tube to the pressure in the surgical cavity as it is assumed the gases path is a sealed path.

### Examples of Tube-set Based Pressure Sensing

A pressure sensor, such as the pressure sensor 56 of Figure 1B, can be included in a secondary pressure line to a gases delivery conduit. The secondary pressure line can be configured to measure pressure of the gases in the surgical cavity, for example, by measuring the pressure inside the gases delivery conduit (which can include any conduit or portions thereof in the gases flow path). The secondary pressure line may not, in some cases, be used for delivery or removal of gases to the surgical cavity.

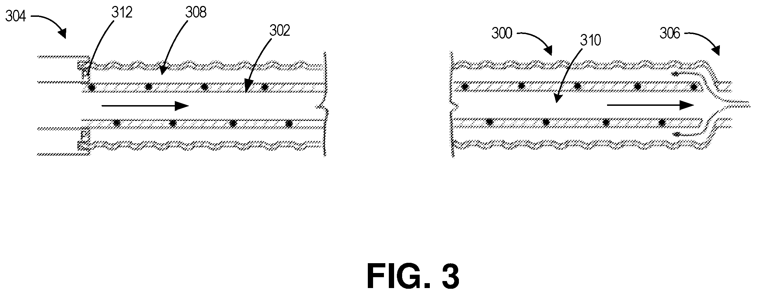

Figure 3 illustrates a cross-sectional view of an example secondary pressure line 300. The secondary pressure line 300 can be co-axially attached or otherwise operably connected to the gases delivery conduit 302. The gases delivery conduit 302 includes a lumen 310, which is an inner lumen, for delivering gases to a surgical cavity. The gases delivery conduit 302 and the secondary pressure line 300 can be coupled to the gases supply at a first end 304 and to the cannula at a second end 306. As described above, a lumen 308, which is an outer lumen, of the secondary pressure line 300 may not be used for delivery or removal of gases as the lumen 308 does not have a gases connection. No therapy gases travel through the lumen 308 to the surgical site. The lumen 308 can be used to determine pressure in the surgical cavity. A pressure sensor 312 can be located at or near the first end 304 of the secondary pressure line 300. The pressure sensor(s) 312 passively measure pressure within the surgical cavity. Some the gases from the surgical cavity may enter the lumen 308 due to the pressure differential between the surgical cavity and the lumen 308, where sensors measure the pressure.

When gases are delivered from the first end 304 to the second end 306, as illustrated by the arrows, the lumen 310 can be pressurized, which can in turn pressurize the surgical cavity. The pressure can be detected by the pressure sensor 312 at the first end 304. The pressure sensor 312 can be located anywhere along the conduit 300.

As shown in Figure 3, the secondary pressure line 300 is coaxial with the gases delivery conduit 302. The secondary pressure line can also be separate from the gases delivery conduit and attach to the cannula and/or the gases supply adjacent to the gases delivery conduit. Adjacent means the secondary pressure line and the gases delivery conduit outlets may be aligned and next to each other. The conduit that includes the gas delivery conduit and the secondary pressure line may all be incorporated into a coupler that connects to a cannula or other surgical port. The lumens of the gases delivery conduit and the secondary pressure line can couple to opposing regions or sides of the cannula, for example, with the secondary pressure line being coupled to a first region and the gases delivery conduit being coupled to a second region that is opposite the first region in the cannula.

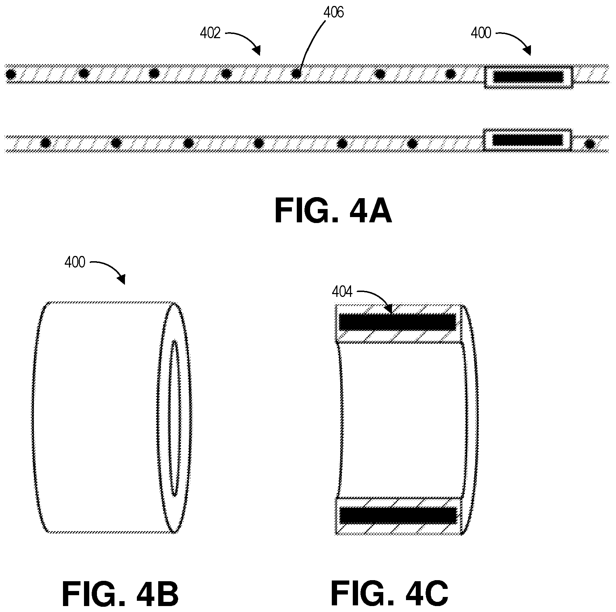

The pressure sensor can be in the form of an expansion ring 400, such as shown in Figures 4A-4D. The expansion ring 400 can be located along and co-axial with the gases delivery conduit 402. The expansion ring 400 can be made of a material that expands and contracts depending on the pressure in the medical gases delivery system. The expansion and contraction can be indicative of the pressure in the surgical cavity. The amount of deformation of the expansion ring 400 and its relation to pressure can be calibrated so that amount of deformation of the expansion ring 400 can be used to determine the pressure of the surgical cavity. As shown in Figure 4C, the expansion ring 400 can include a strain sensor 404 inside a wall of the expansion ring 400 along a length of the expansion ring 400. The strain sensor 404 can detect the amount of deformation in the expansion ring 400, such as due to insufflation of the surgical cavity. The deformation information can be sent to a processor, which can be part of the controller such as the control system 52 of Figure 1B, via an electrical wire or wirelessly. The electrical wire may be the heater wire 406 or a second wire spirally wound next to the heater wire 406 of the gases delivery conduit. The pressure sensors may be wired to or wirelessly coupled to the processor. As alternatives to a heater wire 406, the expansion ring 400 can include any other type of heating element.

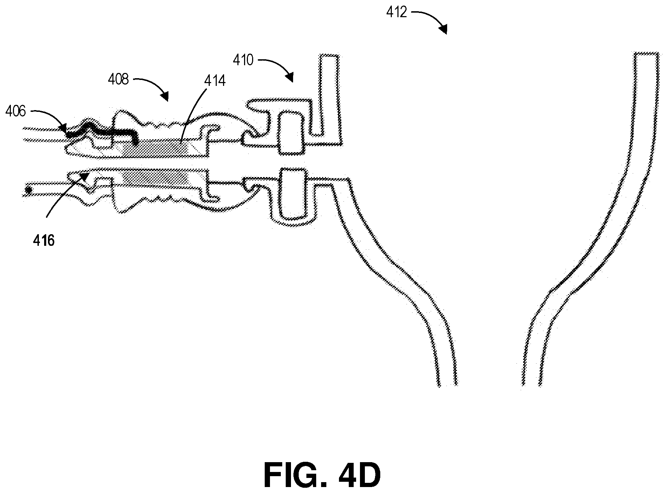

As shown in Figure 4D, the expansion ring can also be mounted to a connector 408 (which can be a barb connector) to a gases port 410 of a cannula 412. The connector 408 can include hand grip features for ease of use when pushing or pulling the connector 408. The connector 408 can include a resilient material, such as rubber, elastomer, or the like. For example, the resilient material of the connector 408 can be overmoulded onto the connector 408. The gases delivery conduit 402 can be connected to the barb 416 of the connector 408 by a push fit or overmoulded onto the barb 416 to secure the gases delivery conduit 402 to the connector 408. The expansion ring 400 can be mounted along and be coaxial with the connector 408. As shown in Figure 4D, the expansion ring 414 can be integrated into the connector. The amount of deformation of the expansion ring 414 can be used to determine the pressure of the surgical cavity. In other words, deformation of the ring 414 is indicative of the pressure in the surgical cavity. The expansion ring 414 may be similar to the expansion ring 414 described earlier. The expansion ring may be incorporated within the gases pathway or around the barb connector under the resilient material. Alternatively, the expansion ring may be embedded within the resilient material. The heater wire 406 is helically wound in the illustrated example. The heater wire 406 or any other type of suitable heating element can be in electrical communication with the expansion ring 414. Accordingly, the heater wire 406 or any other type of suitable heating element can send signals of the deformation back to the processor in the humidifier and/or at the gases source for pressure calculations.

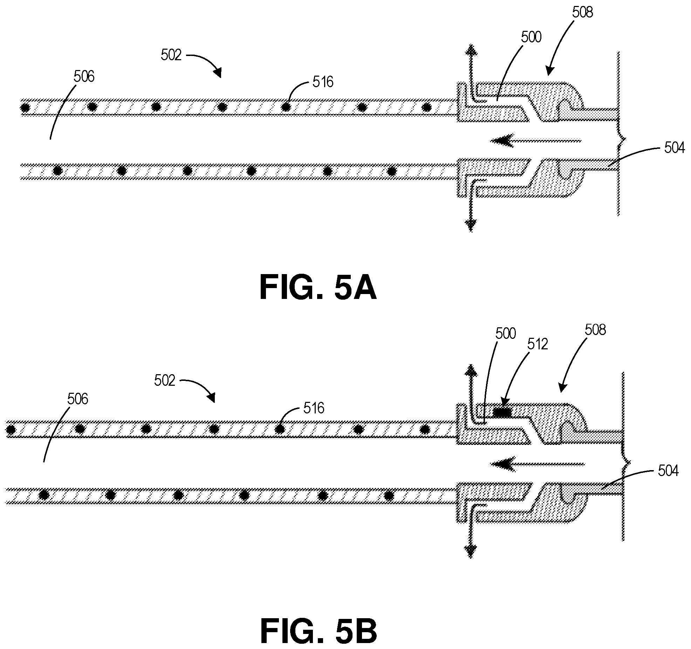

A controlled leak via an orifice known to the system can be used to detect pressure in the medical gases delivery system. As shown in Figures 5A-5C, an orifice 500 known to the system can be located at a connector 508 between the gases delivery conduit 502 and an outlet 504 of the gases supply. The orifice 500 can be formed into the connector 508 such that the orifice 500 provides a leak path for gases from the surgical site. As the gases travel from the outlet 504 into a lumen 506 of the gases delivery conduit 502, gases can escape through the orifice 500. The proportion of gases leaking vs. entering the lumen 506 can be based on a ratio of cross-sectional areas of the orifice 500 and the connector 508. The leak through an orifice of a known size in the connector, in addition to the provided flow and/or provided pressure is used to determine pressure in the surgical cavity. The leak through the orifice 500 can change based on a driving pressure from the pneumoperitoneum and/or gases supply which can be correlated to the pressure inside the surgical cavity. For example, the known orifice has a known relationship between a flow through the orifice and a pressure to cause the flow rate through the orifice. The leak rate, or leak velocity, can be measured using a flow (for example, a thermal mass flow) sensor 512 or any other type of sensor that would detect flow rate or flow velocity. The flow rate from the gases supply is used in addition to the flow rate through the orifice (or the leak rate) can be used to determine the pressure in the surgical cavity.

In Figure 5A, the leak rate or leak velocity can be measured using a thermal mass flow sensor. The thermal mass flow sensor can include a heating element that uses anemometry to determine the velocity of the gases from heat loss due to convection. The sensor may be a hot wire anemometer that is used to determine the velocity of gases through the orifice. The velocity is determined based on measuring heat loss of the wire that is placed in the fluid path of the orifice 500 such that the leak flow passes over and across the wire of the anemometer. The wire can be heated by power from a supply line. For example, the heater wire 516 may be extended or an electrical line may be extended from the heater wire 516 to connect to the anemometer to provide power to the wire in the anemometer.

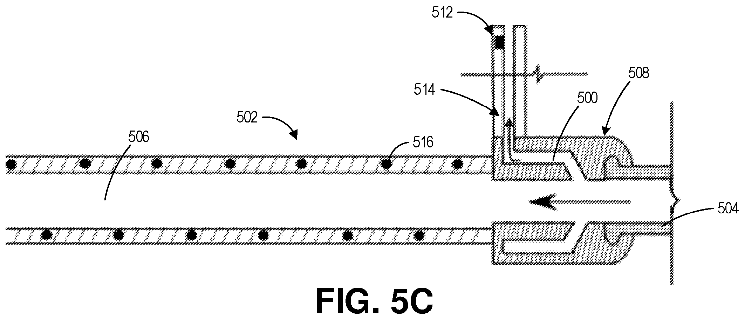

As shown in Figure 5B, the connector 508 can also include a flow sensor 512 in fluid communication with the orifice 500 to measure the leak rate. As shown in Figure 5C, the flow sensor 512 can also optionally be incorporated or integrated into a Luer connector or a leak tube 514 coupled to an outlet of the orifice 500 via the Luer connector. The leak tube 514 can be a different tube than the gases delivery tube 502. The leak tube 514 can terminate at an open end that is open to atmosphere so that the flow sensor 512 can measure a leak rate. Alternatively, the leak tube 514 can be coupled to a suction unit on an opposite end from the outlet of the orifice 500. The leak tube 514 can also be coupled with a filter that filters smoke, particulates, and/or the like.

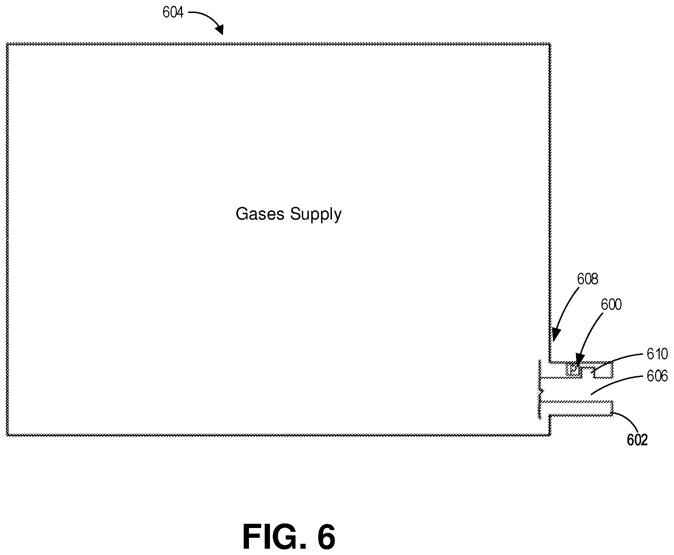

Figure 6 illustrates an example pressure tap 610 with a pressure sensor 600 in communication with an insufflator 606. For example, the pressure sensor 600 can be located on a connector 602 of the insufflator 606 to which a tube attaches. The pressure tap 610 can be integrally built into, or later attached to the connector 602. The pressure sensor 600 can be directly in the gas flow path. The pressure tap 600 can draw some gases, for example, air from the tube 606 into the tap 610 to allow the pressure sensor 600 to read the pressure of the gases in the tube. The pressure sensor 600 reads the pressure of the gases being delivered. The system of Figure 6 operates on the assumption that the surgical cavity is substantially sealed and pressure in the gases delivery conduit is equal to the pressure in the surgical cavity. The processor considers the pressure reading at the sensor 600 to be equal to the pressure in the surgical cavity based on this assumption. Alternatively, the processor may know the leak from the surgical cavity or may use a pre-programmed known leak flow rate that is used to calculate the pressure in the surgical cavity based on the measured supply pressure and the known leak. The pressure tap 610 can also be incorporated into a gas manifold at (or near) an outlet 608 of the gases supply 604 (or a humidifier outlet or inlet). The pressure sensor can determine pressure in the tube 606 and hence pressure inside the surgical cavity.

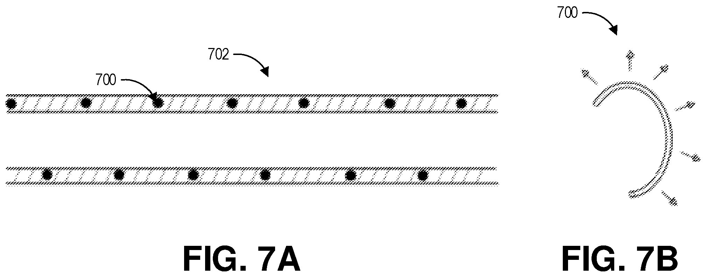

Pressure sensing can be performed by monitoring an amount of deformation of the heater wire in or on the wall of the gases delivery conduit. As shown in Figure 7A, a heater wire 700 can be helically wound along the wall of the gases delivery conduit 702. As shown in Figure 7B, the heater wire 700 can deform radially in response to a pressure change of the medical gases delivery system. The pressure changes may be due to the delivered pressure and pressure changes in the cavity. Deformation of the heater wire 700 can change an electrical resistance of the heater wire 700. The change in electrical resistance, measured via a sensor, for example, an electrical resistance sensor or a transient current detector, can be used to determine the pressure of the surgical cavity by determining the relationship of the resistance change to pressure change. Alternatively and/or additionally, a change in the inductance can be measured. The relationship between the resistance change and the pressure change in the conduit is determined experimentally for a particular type of conduit and the heater wire type. A variety of delivery conduits with different heater wires can be integrated into the wall of the conduit. One non-limiting example is the AirSpiral^{™} tube from Fisher & Paykel Healthcare Limited (Auckland, NZ). Tubes like the AirSpiral^{™} tube can be experimentally characterized, for example, the relationship between the resistance change and the pressure change can be determined. The relationship can be stored in the processor, which may be part of the controller, and can be used to determine pressure in the tube based on the measured heater wire resistance change. The pressure in the tube is equated to the pressure in the surgical cavity based on the assumption of a sealed pathway from the insufflator to the surgical cavity. The processor may also store a relationship of a known leak of the surgical cavity. The known leak can be used to further determine the pressure in the surgical cavity.

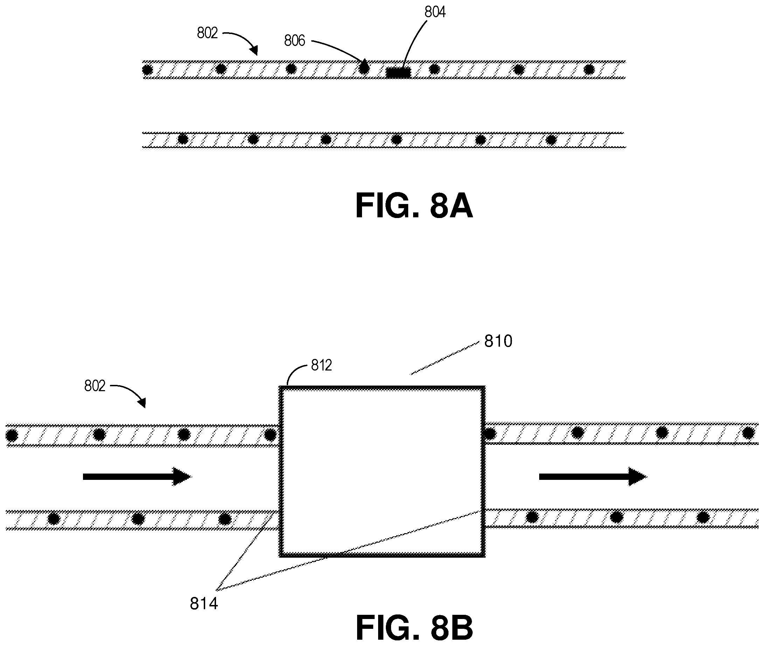

Figure 8A illustrates a flow sensor 804 that is in-line with a tube 802, which can be a gases delivery tube. The flow sensor 804 can be any known flow sensor, such as being standard, off-the-shelf, or the like. The flow sensor 804 can be integrated into the wall of the tube 804, or secured in place so that the flow sensor 804 is exposed to the gases flow path in the tube 802. The flow sensor 804 can be in wireless communication with the processor in the humidifier and/or gases supply. The flow sensor 804 can also have a wired connection, for example, with the heater wire 806 in the tube 802 so that the heater wire 806 can act as a signal carrier for sending the flow signal to the processor.

As shown in Figure 8B, a modular flow sensor 810 incorporating any of the flow sensors and/or methods disclosed herein, and/or any other suitable flow sensing methods, can be connected to a tube 802, such as a gases delivery conduit, or any portion of a gases supply tube set. The modular flow sensor 810 can be incorporated into a housing or unit 812, which can be connected to the tube 802 or the gases supply tube set. The tube(s) can be integrated into the unit 812 (for example, via overmoulding, a barb connector, or otherwise) or have removable connections (via Luer connectors, snap-fit connectors, or otherwise) for connecting to the unit 812 at connection points 814.

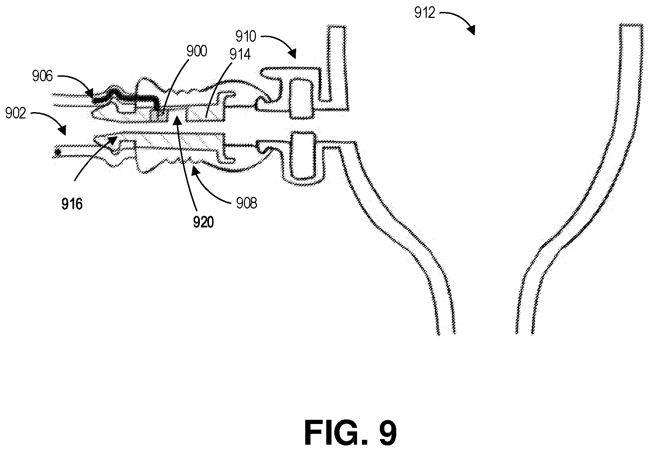

As shown in Figure 9, a pressure tap 920 with a pressure sensor 900 can also be located near a gases port 910 of a cannula 912. The pressure tap 920 can have any features of the pressure tap 610 described above. The pressure tap 920 is a small passageway that allows the pressure sensor 900 to sense the pressure in the gases pathway and thus the pressure in the surgical cavity based on the assumption that the pressure in the gases pathway of the connector is approximately equal to the pressure in the cavity. Alternatively, the processor may operate using a preprogrammed value of a known leak through the surgical cavity. The pressure reading from the pressure sensor 900 and the known leak rate can be used to calculate the pressure in the surgical cavity.

The pressure tap 920 can be located in the connector 908, which can have any features of the connector 408 described above, coupled to the gases port 910 (for example, via a Luer connection or other types of connection). The pressure tap 920 can be built into the connector 908, for example, within the barb connector 914 including barb 916. The pressure sensor 900 can be directly in the gases flow path. The pressure tap 920 can also be incorporated into a gas manifold at (or near) the gases port 910. The pressure sensor 900 can determine pressure in the gases delivery conduit 902 and hence pressure inside the surgical cavity. The pressure sensors disclosed herein are configured to transmit electrical signals that are processed by a processor to determine the pressure measured by the sensor. The electrical signal may be a signal relating to voltage or current. The heater wire 906 of the gases delivery conduit 902 can be in electrical communication with the pressure sensor 900 and can send signals from the pressure sensor 900 back to the gases supply processor for pressure calculations.

In other configurations, the conduit may comprise additional sensor wires. The sensor wires may be embedded into the wall of the conduit and extend along the length of the conduit. Alternatively, the sensor wires may be positioned in the lumen of the conduit. The sensor wires are configured to transmit signals from the sensor to the processor for processing the sensor signals. The sensor wires may also be configured to transmit power signals to the pressure sensor to power the sensor. Generally, the tubes disclosed herein that incorporate using the heater wire to transmit sensor signals can include separate heater wires and separate sensor wires to transmit signals. The sensor signals may be read at the zero crossing of the power signals, wherein the power signals are DC signals.

For all configurations disclosed herein, the pressure sensor may be configured for wired communication of sensor signals, or alternatively may be configured for wireless communication with the processor.

The pressure sensor or pressure sensing apparatus can be integrated into a component of the tube set and isolated from the gases supply to reduce any contamination risk. The tube set may be reused in some uses, and the pressure sensor or pressure sensing apparatus being located in the tube set reduces the need to constantly replace the pressure sensor or pressure sensing apparatus. A pressure sensor or pressure sensing apparatus in the cannula requires the pressure sensor or pressure sensing apparatus to be discarded with the cannula, which is discarded after a surgical procedure. Further, including the pressure sensor or pressure sensing apparatus in the tube set can advantageously move the pressure sensor away from any instruments inserted into the cannula, thereby reducing the chances of electronic interference or damage from the instruments to the pressure sensor or sensing apparatus.

### Examples of Cannula Based Pressure Sensing

Pressure inside the cannula can be determined so as to determine the pressure inside the surgical cavity. The pressure inside the cannula can be measured by a pressure sensor or sensing apparatus incorporated into the cannula. The pressure sensor or sensing apparatus may be incorporated into a portion of the cannula and provide an indication of the pressure in the surgical cavity.

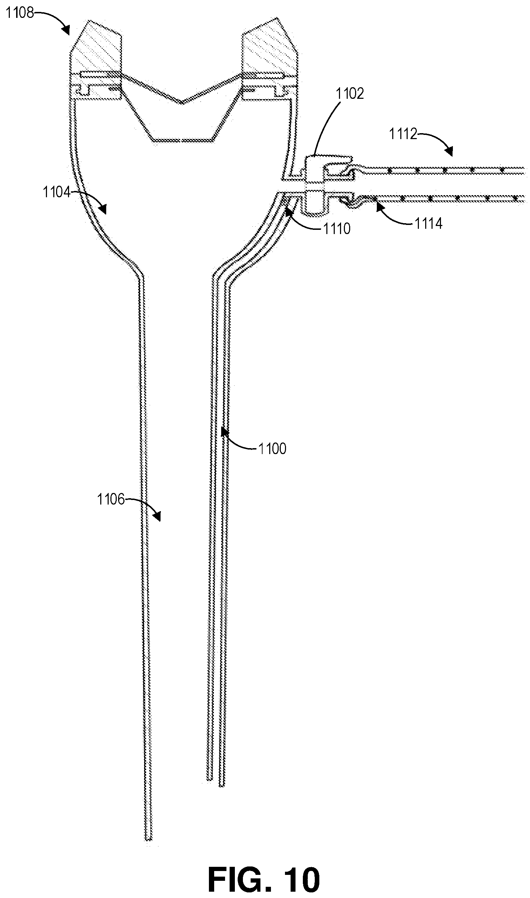

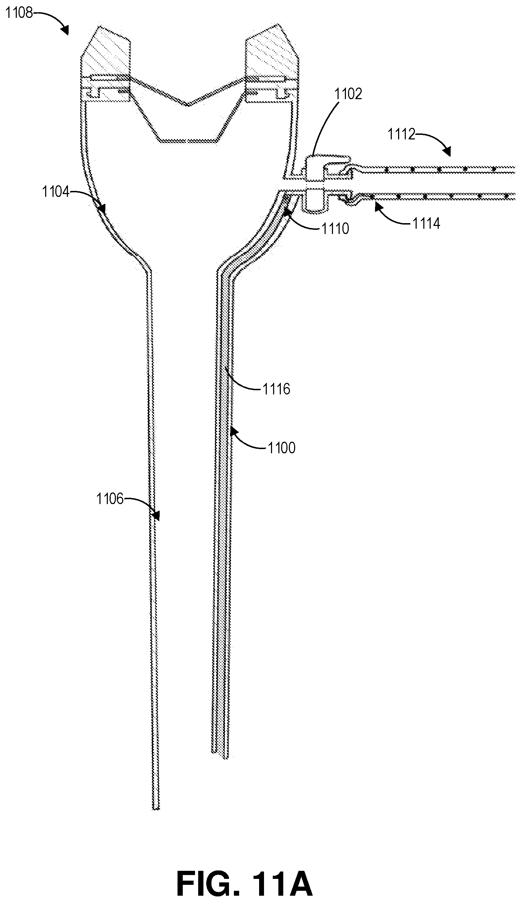

The present disclosure provides examples of a pressure sensing channel (Figures 10 and 11A-11B) built into the gases delivery cannula, which is directed into the surgical cavity. The pressure sensing channel can allow pressure measurement of the surgical cavity directly. As shown in Figure 10 and 11A-C, the pressure sensing channel 1100 can extend from the gases port 1102 along portions of the body 1104 (which is generally removable from the cannula) and the shaft 1106 of the cannula 1108. The pressure sensing channel 1100 allows gases from the surgical cavity to equalize across the channel 1100 such that the channel 1100 is pressurized to the same pressure as the pressure in the surgical cavity. The pressure sensor 1110 senses the pressure in the channel 1100. In particular, the pressure sensor 1110 generates one or more signals indicative of pressure in the channel 1100, which is equated to the pressure in the surgical cavity. The processor is configured to process the signals from the pressure sensor 1100 and determine an actual pressure value. A pressure sensor 1110 can be located at an end of the pressure sensing channel 1100 near the gases port 1102 to determine the pressure inside the surgical cavity. The gases delivery conduit 1112 can provide power to the pressure sensor 1110. The gases delivery conduit 1112 may include one or more sensor wires that are configured to provide power to the pressure sensor 1110 and/or transmit sensor signals to the processor for processing. In one preferred example construction, the wire includes a pair of sensor wires that are used to power the pressure sensor 1110 and also transmit signals from the sensor to the processor. The gases delivery conduit 1112 may also include additional heater wires 1114. In an alternative configuration, the heater wire 1114 of the gases delivery conduit 1112 transmits power to the pressure sensor 1110. For example, the heater wire 1114 in the gases delivery conduit 1112 can be connected to the pressure sensor 1110 upon the gases delivery conduit 1112 being connected to the gases port 1102 to provide power to the pressure sensor 1110. In an alternative configuration, the sensor wires may simply provide power and sensor readings, that is, sensor signals, can be wirelessly transmitted to the processor.

Figures 11A-11C illustrate a medium 1116 contained within the pressure sensing channel 1100. The medium 1116 can fill substantially an entire internal space of the pressure sensing channel 1100. The medium 1116 can act in a measurable manner over the pressure range that the surgical cavity is known to commonly be subjected to. For example, the medium 1116 can deform (such as expanding, shrinking, curving, etc.) in response to pressure changes in the surgical cavity. The pressure in the surgical cavity can be determined by monitoring the deformation of the medium 1116. The medium may be a fluid, in particular, a viscous fluid that is configured to transmit pressure in the surgical cavity through the channel 1100 to the pressure sensor 1110. The pressure sensor 1110 is configured to measure the changes in the pressure and transmit electrical signals indicative of the pressure in the channel 1100. A processor is configured to process the signals and determine a pressure in the channel 1100. The pressure in the channel is assumed to be equivalent to or indicative of the pressure in the surgical cavity.

In an alternative configuration, the medium 1116 may be a solid material, for example, a foam or a flexible plastic. In some configurations, the sensor may be a strain gauge, a force sensor, or a strain sensor. The solid material of the medium 1116 is configured to change its physical characteristics in response to changes in the pressure within the channel 1100 due to changes in the pressure in the surgical cavity. The changes in the physical characteristics can be related to the pressure in the channel 1100 by a mathematical function. The controller or the processor may be configured to store the mathematical function and utilize the mathematical function to determine the pressure in the surgical cavity.

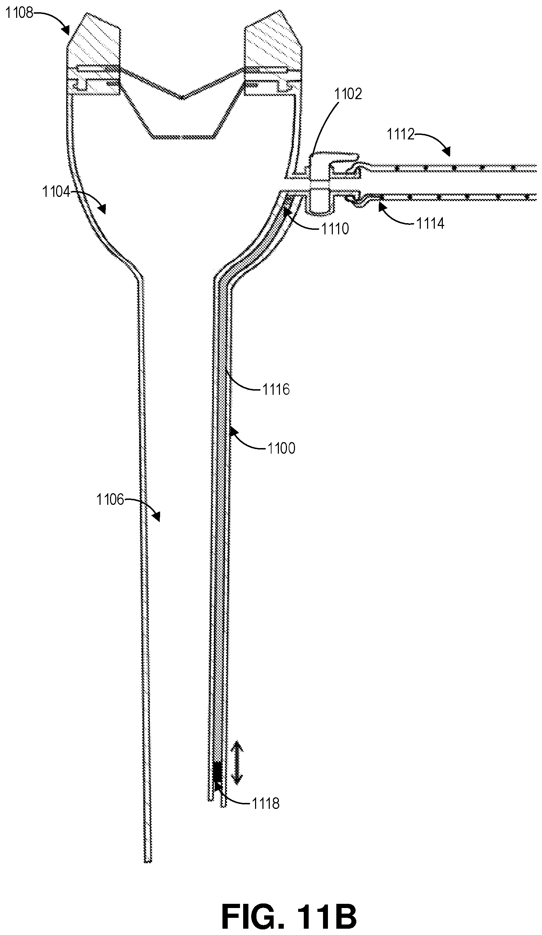

In Figure 11A, the medium 1116 is open to the surgical cavity environment. In another configuration, such as shown in Figure 11B, the cannula 1108 may include a slidable plug 1118 in the pressure sensing channel 1110. The slidable plug 1118 can pneumatically seal the medium material 1116 from the surgical cavity environment. The slidable plug 1118 is free to move up and down along the pressure sensing channel 1110. The sliding movements of the plug 1118 can be based on the pressure in the surgical cavity. Movements of the slidable plug 1118 can transfer the pressure in the surgical cavity into the medium 1116, which may be solid or fluid as described above. The slidable plug 1118 can slide along an inner wall of the pressure sensing channel 1100 using lubricated surfaces, on a linear translation mechanism (for example, one or more rails), or any other method that can allow linear translation of the slidable plug 1118 with changes in pressure in the surgical cavity. The slidable plug 1118 can visually indicate the pressure of the surgical cavity (for example, by having gradations along the pressure sensing channel 1100. Alternatively or additionally, the cannula includes a sensor (such as an electrical resistance sensor or otherwise) to track the position of the slidable plug 1118 relative to a reference position.

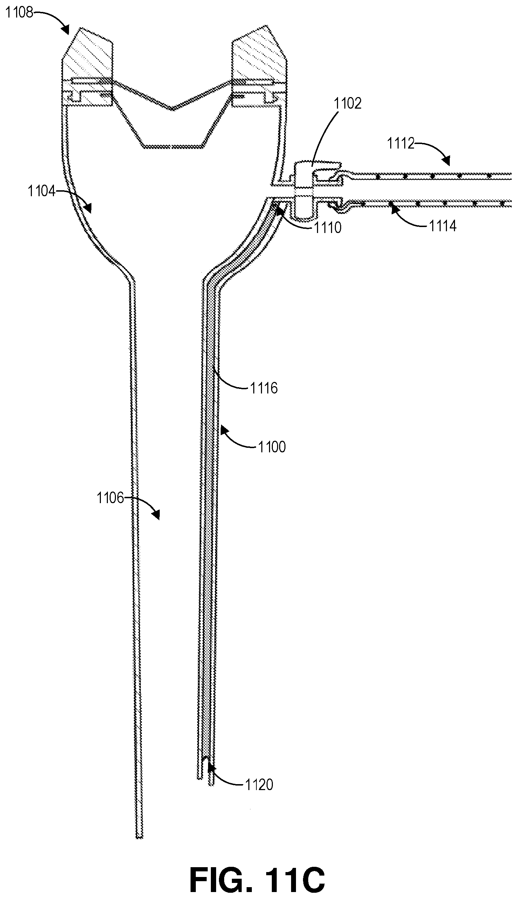

A further alternative configuration, such as shown in Figure 11C, the cannula 1108 can include a diaphragm 1120 instead of the plug 1118 of Figure 11B. The diaphragm 1120 can be fixedly attached to an inner wall of the pressure sensing channel 1110. The diaphragm 1120 can be integrated into the inner wall of the pressure sensing channel 1110 (for example, using overmoulding), or can be secured/fastened in place using fastening mechanisms (for example, adhesives). The diaphragm 1120 can pneumatically seal the medium 1116 (which can be solid or fluid as described above) from the surgical cavity environment. The diaphragm 1120 can include a flexible material that is free to deform up and down (or change in degree of concavity and/or convexity) in response to pressure changes, which in turn transfers the pressure of the surgical cavity into the medium 1116, causing the medium 1116 in the channel 1110 to move or deform. The cannula 1108 can include a sensor, for example, a force sensor or a strain sensor to determine the change in the physical characteristic of the medium 1116 as described above.

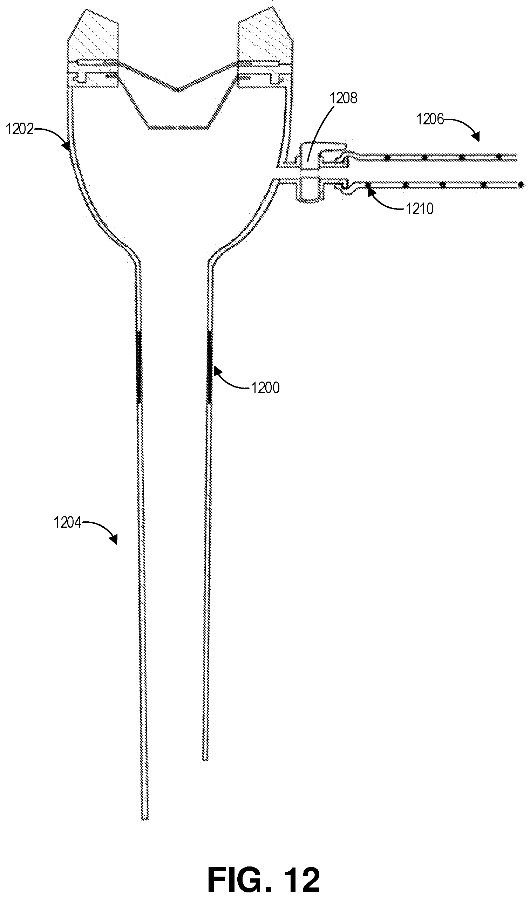

Figure 12 illustrates an element 1200 that deforms under pressure changes, for example, at least under the pressure ranges of the surgical cavity. The element 1200 can be a strain gauge. The amount of deformation of the element 1200 can be proportional to the pressure or pressure change in the surgical cavity. As shown in Figure 12, the element 1200 can be located on or within the shaft 1204 of the cannula 1202. The element 1200 can be overmoulded into the wall of the shaft 1204. The deformation of the wall of the cannula can be used to determine the pressure inside the surgical cavity. The gases delivery conduit 1206 can provide power to the element 1200. For example, the heater wire 1210 can be connected to a wire that extends from the element 1200, upon the gases delivery conduit 1206 being connected to the gases port 1208 to provide power to the element 1200. The strain gauge 1200 may be overmoulded onto the wall of the shaft 1204. The relationship between pressure in the surgical cavity and the deformation of the shaft 1204 can be determined experimentally.

The relationship between the pressure and deformation can be used by the processor to determine the pressure in the surgical cavity based on the strain gauge measurement provided by the element 1200 in the form of a strain gauge. The strain gauge provides a signal that is indicative of a deformation, that is, strain experienced by the shaft 1204. The processor is configured to determine the strain based on the strain gauge signal and then determine a corresponding pressure in the surgical cavity based on the mathematical relationship between deformation and pressure. The cannula in Figure 12 may include additional sensor wires that couple the strain gauge 1200 to the processor to communicate the signals from the strain gauge to the processor. The sensor wires may be integrated into the wall of the shaft 1204 in addition to heater wires (if present). Alternatively, the strain gauge may communicate wirelessly the electrical signals indicative of strain to the processor.

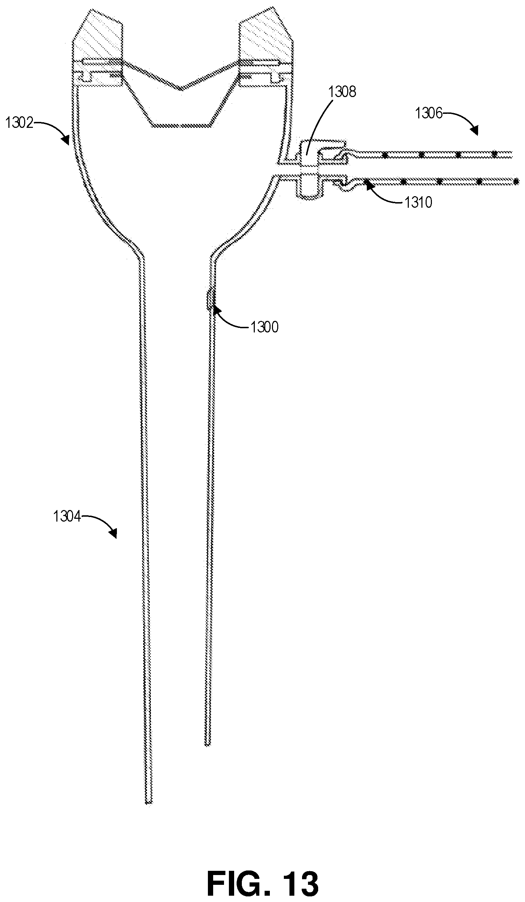

The medical gases delivery system can be configured such that the flow rate of the gases flow inside the cannula 1302 (at least in the shaft 1304) is constant. As shown in Figure 13, a flow rate sensor 1300 (such as a heated thermistor or otherwise) can be located in the gases flow in the shaft 1304 of the cannula 1302. The flow rate sensor 1300 can be built into the wall of the shaft 1304. The continuous flow rate in the cannula 1302 and the measurements from the flow rate sensor 1300 can be combined to determine the pressure of the surgical cavity. The pressure in the cannula is determined based on the measured flow value. The flow rate measured corresponds to the flow rate from the insufflator. The continuous flow rate may be a constant flow rate or may be a cyclic flow rate above 0. Preferably, the flow rate is always kept above 0. The measurements from the flow rate sensor 1300 can be used to estimate the pressure of the surgical cavity based on either the gases flow into the patient or the gases flow out of a known leak as described above. The pressure in the surgical cavity may be determined based on a mathematical relationship between the flow rate and the pressure in the cannula. In one example, Bernoulli's equation can be used to determine the pressure in the cannula and hence the pressure in the surgical cavity. Bernoulli's equation relates the pressure and the flow rate. Alternatively, the relationship between the flow rate and the pressure may be experimentally determined for the illustrated cannula example. A processor of the controller uses one of these methods to determine the pressure in the cannula. The pressure in the cannula is assumed to be the same as the pressure in the surgical cavity.

The gases delivery conduit 1306 can provide power to the flow rate sensor 1300. For example, the heater wire 1310 can be connected to a wire that extends from the flow rate sensor 1300, upon the gases delivery conduit 1306 being connected to the gases port 1308 to provide power to the flow rate sensor 1300. The cannula in Figure 13 may include additional sensor wires that couple the flow rate sensor 1300 to the processor to communicate the signals from the sensor 1300 to the processor. The sensor wires may be integrated into the wall of the shaft 1304 in addition to heater wires (if present). Alternatively, the sensor 1300 may communicate wirelessly the electrical signals indicative of strain to the processor.

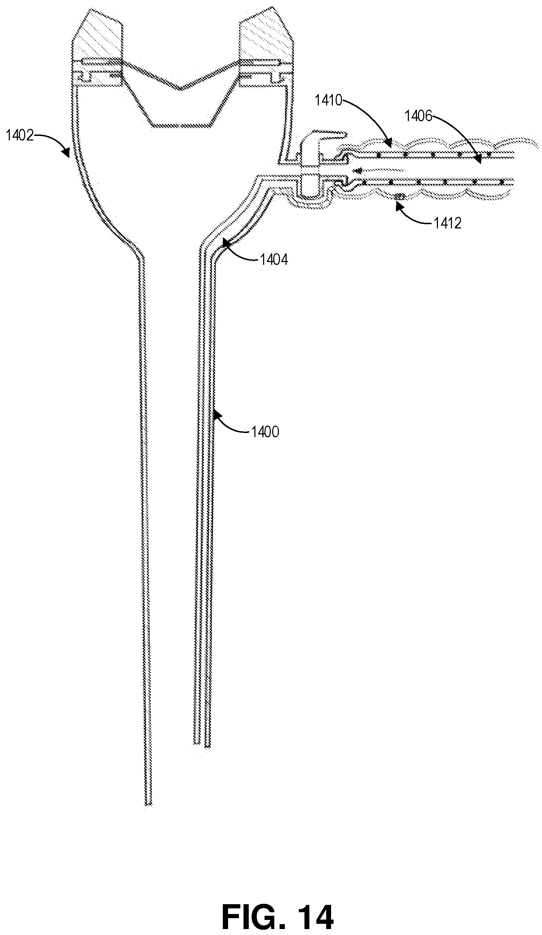

As shown in Figure 14, the cannula 1402 can have a pressure channel 1400 having any of features of the pressure channel 1100 described above, except the pressure channel 1400 does not include any sensor or medium. The pressure channel 1400 can have an internal space 1404 that is in fluidic communication with a lumen of a secondary pressure sensing line 1410, such as the secondary pressure sensing line 300 described above. For example, the secondary pressure sensing line 1410 can be substantially coaxially attached to the gases delivery conduit 1406. A pressure sensor 1412 located in the secondary pressure sensing line 1410 can measure pressure of the gases flow upstream of the cannula 1402. The pressure sensor 1412 can be located in the conduit coupled to the gases supply or within the gases supply (such as at the outlet of the gases supply). The readings of the pressure sensor 1412 can be used to estimate the pressure inside the surgical cavity. In one example, the pressure measured by the pressure sensor 1412 is assumed to be the pressure in the surgical cavity since the surgical cavity, the cannula 1402, and the gases delivery conduit 1406 that is coupled to the insufflator are assumed to be a sealed system within minimal leak.

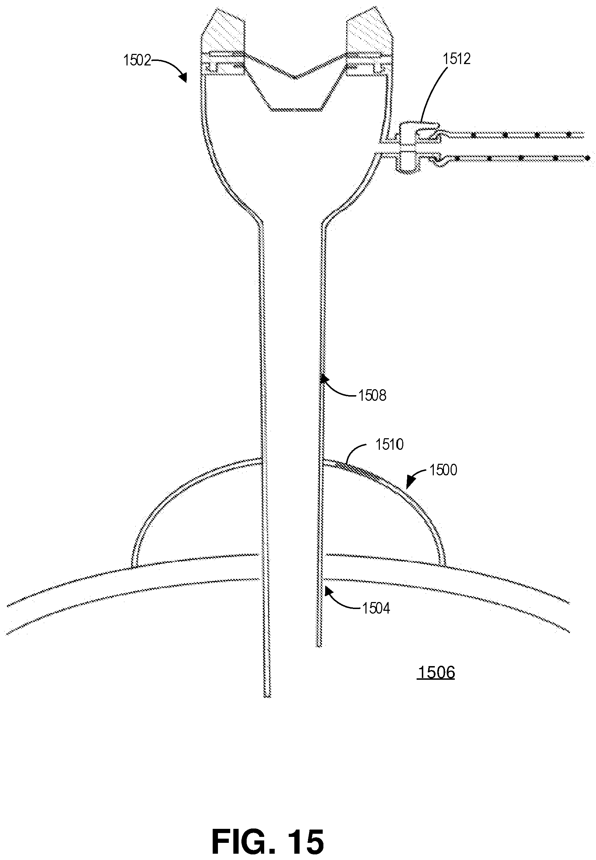

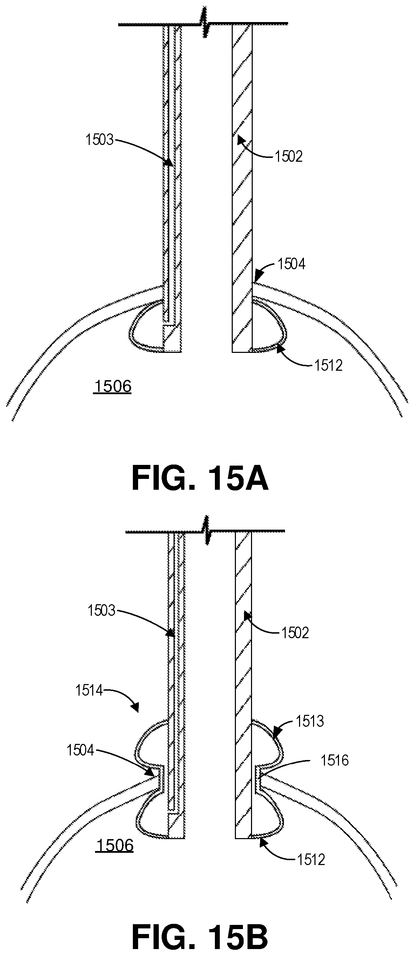

As shown in Figure 15, the cannula 1502 can be inserted into the surgical cavity 1506 at an incision site 1504. A sleeve 1500 can surround the cannula 1502 and the incision site 1504, forming a sealed or airtight environment around a portion of the cannula shaft 1508 and the incision site 1504. For example, the sleeve 1500 can attach to the patient's skin (such as via adhesives or otherwise) to fully enclose the incision site. The sleeve 1500 can have a concave shape like an opened umbrella. Pressure within the sleeve 1500 can develop (that is, the sleeve 1500 inflates) from leaks around the incision site 1504. The pressure inside the sleeve 1500 is equal to or substantially equal to the pressure inside the surgical cavity 1506. A strain sensor 1510 or any other pressure sensor can be located on the sleeve 1500. The strain sensor 1510 can deflect in an amount that relates to the pressure inside the surgical cavity. A predetermined relationship between strain and pressure can be used to determine the pressure at a measured strain. The predetermined relationship may be experimentally determined and preprogrammed into the processor.

The wall of the cannula shaft 1508 in the portion surrounded by the sleeve 1500 can include one or more channels for gases to inflate the sleeve or balloon 1500 more quickly, for example, if the normal surgical leaks around the incision site 1504 are insufficient or the rate of inflation of the sleeve 1500 is too slow so the pressure sensing response has too much lag. The tube set can power the strain gauge similar to the arrangement of Figure 12. The strain gauge can be powered by other sources, for example, via flying leads or cables that extend from the cannula 1502 or the connector 1508 of the tube and couple to the strain gauge 1510. Alternatively, the strain gauge may wirelessly communicate with the processor.

For the configuration in Figure 15, there is a mechanical gas path (rather than relying on unintended leaks) between the surgical cavity 1506 and the balloon 1500. The assumption is that gases can flow freely from one of those two locations to the other location with minimal lag. Algorithms can be implemented to trigger a warning if the cannula 1502 has not been inserted properly resulting in an obstructed gas path, for example, resulting in the inflation of the balloon 1500 being too slow.

As an alternative to the sleeve 1500, as shown in Figure 15A, a first retention member 1512 that is located at or near a distal end of the cannula 1502 can be inserted under the incision site 1504, that is, on the side of the surgical cavity 1506. The retention member can be initially deflated during insertion and then can be inflated to create the retention and sealing around the incision site 1504. Inflation can be via a channel 1503 extending from the first retention member 1512 to a port leading to a gas source, which can be separate from the primary gases source delivered to the cannula 1502. The first retention member 1512 can form a seal around the cannula 1502 at the incision site 1504 after inflation of the first retention member 1512. The first retention member 1512 can also secure the cannula 1502 to the surgical cavity 1506. Optionally, a second retention member (not shown in Figure 15A), can be added outside the incision site 1504 as an additional securing member. The second retention member can form a unitary construction with the first retention member 1512 (see for example, Figure 15B), or be separate from the first retention member 1512. The second retention member may also be inflatable. The inflatable second retention member may share the same gas supply as the first retention member, or have its own gas supply separate from the primary gases source and from the gas supply to inflate the first member. Pressure within the first retention member 1512 can build up from leaks around the incision site 1504. The pressure inside the first retention member 1512 can be equal to or substantially equal to, or be correlated with the pressure inside the surgical cavity 1506. A pressure sensor can be located on or in the first retention member 1512, or anywhere in the channel 1503. In some configurations, the pressure sensor can be a strain sensor as described with reference to Figure 15. The retention members can be made of, for example, plastic, silicone, or rubber.

As shown in Figure 15B, a retention unit 1514 that can be of a unitary construction in some cases can include a first retention portion 1512 and a second retention portion 1513. The first retention portion 1512 can be inserted under the incision site 1504 (that is, on the same side as the surgical cavity 1506) and a second retention portion 1513 can be located outside the incision site 1504 (that is, outside the surgical cavity 1506). The retention portions can be initially deflated during insertion and then can be inflated to create the retention and sealing around the incision site 1504. Inflation can be via a channel 1503 extending from the retention unit 1514 to a port leading to a gas source, which can be separate from the primary gases source delivered to the cannula 1502. The incision site 1504 can constrict around a narrower portion 1516 between the first and second portions 1512, 1513. The first and second retention portions 1512, 1513 can secure the cannula 1502 to the surgical cavity 1506. The first and second retention portions 1512, 1513 can be in fluid communication with each other. Pressure within the retention unit 1514 can build up from leaks around the incision site 1504. The pressure inside the retention unit 1514 can be equal to or substantially equal to, or correlated with the pressure inside the surgical cavity 1506. A pressure sensor can be located on or in the retention unit 1514, for example, on or in the first retention portion 1512 or on or in the second retention portion 1513, or anywhere in the channel 1503. In some configurations, the pressure sensor can be a strain sensor as described with reference to Figure 15. The retention portions can be made of, for example, plastic, silicone, or rubber. Alternatively, a first retention portion and a second retention portion can be separate components, with the pressure sensor being located in the first retention portion that is underneath the skin.

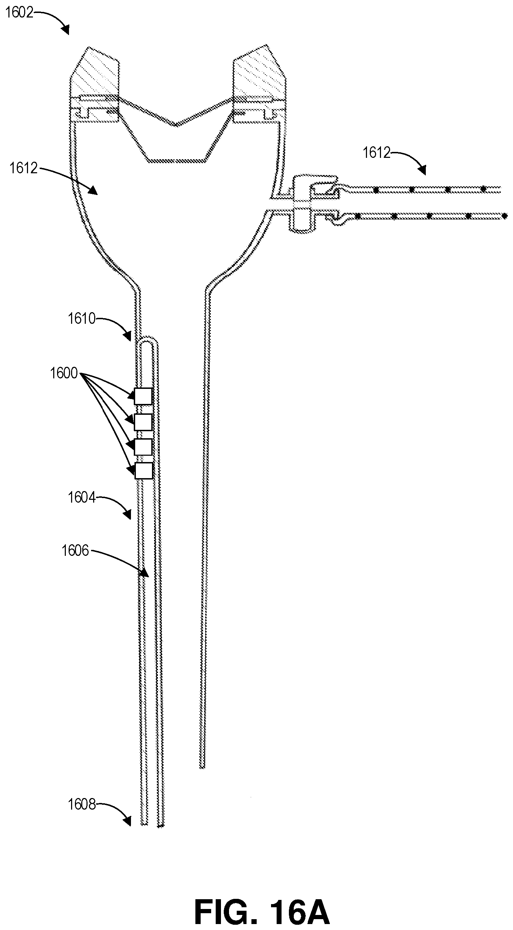

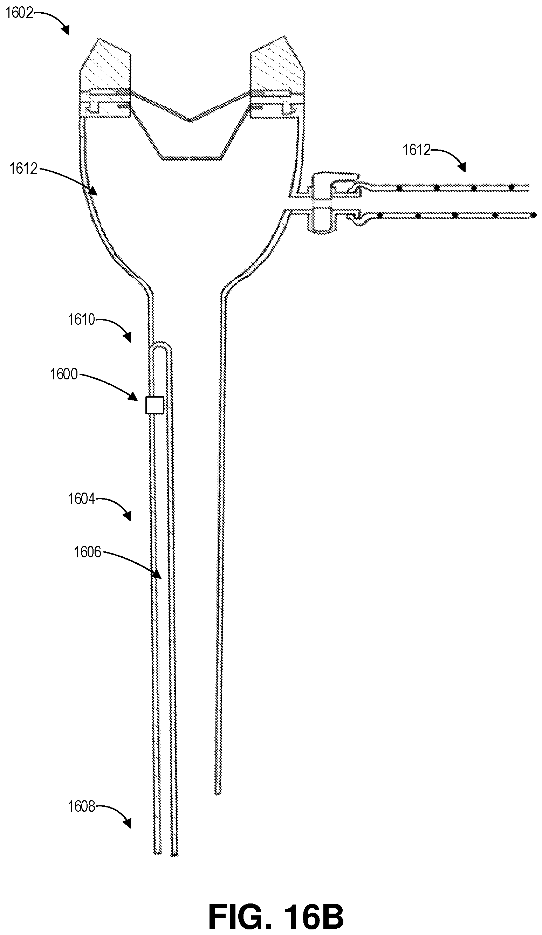

Figures 16A and 16B illustrates valves on a cannula for pressure sensing and/or control. As shown in Figures 16A and 16B, a pressure channel 1606 is located within the cannula shaft 1604 of the gases delivery cannula 1602. The outer wall of the cannula shaft 1604 and the outer wall of the pressure channel 1606 can overlap at least partially. The pressure channel 1606 can have an open end 1608 at a distal end of the cannula shaft 1604 configured to be inserted into and in fluidic communication with the surgical cavity. The pressure channel 1606 can have a closed end 1610 opposite the open end. The closed end 1610 can be located nearer to the cannula body 1612 than the open end 1608.

As shown in Figure 16A, the pressure channel 1606 can include one or more pressure-indicating valves 1600 (for example, mechanical valves that open when the pressure in the channel 1606 reaches a certain threshold) in the pressure channel 1606. The one or more pressure-indicating valves 1600 can be configured to open at predetermined pressure values. The pressure inside the channel 1606 is indicative of the pressure in the surgical cavity. The valve 1600 may include a flag or other indicia that provides a visual indication of the valve 1600 being activated. The one or more pressure-indicating valves 1600 can provide a visual and/or audible indication in addition to an electronic signal being generated once a valve 1600 is opened or activated. A processor can determine, based on the electronic signal, the specific valve that is activated to determine pressure in the cannula, and thereby equate it to the pressure in the surgical cavity. The pressure-indicating valves 1600 can also be implemented with pilot valves. When any one of the pressure-indicating valves 1606 is open, a small amount of gases in the medical gases delivery system, such as in the cannula shaft 1604 and/or the surgical cavity, may be leaked from the system while the pressure inside the surgical cavity is maintained.

As shown in Figure 16A, the pressure channel 1606 can accommodate a plurality of pressure-indicating valves 1600 on the outer wall of the pressure channel 1606 along a length of the cannula 1602. The pressure-indicating valves 1600 can be graduated so that each pressure-indicating valve 1600 can open based on specific and different pressures. For example, the valve 1600 can open at different pressure intervals from about 0 mmHg to about 20 mmHg or from about 0 mmHg to about 40 mmHg, or at about, at least about, or no more than about 5, 10, 15, 20, 25, 30, 35, 40mm Hg, or ranges including any two of the foregoing values. Other pressure ranges are also contemplated. The activation pressure of each valve is also predetermined and each valve used can have a particular set pressure. Some pressure-indicating valves 1600 can open at a higher pressure than a remainder of the valves. For example, the pressure-indicating valves 1600 closer to the open end 1608 can be configured to open at a lower pressure than the pressure-indicating valves 1600 closer to the closed end 1610. A sensor coupled to the processor is used to monitor which pressure-indicating valves 1600 opens, which indicates the pressure of the surgical cavity. Alternatively, each valve 1600 may be electrically coupled to the processor to allow the processor to determine which valve 1600 has been activated and/or how many valves 1600 have been activated. For example, at a low pressure, a first valve 1600 may be activated. At a higher pressure, multiple valves 1600 may be activated. The processor can detect opening of one or more valves 1600 and determine the pressure corresponding to the valve or valves 1600 that are activated.

As shown in Figure 16B, the pressure channel 1606 can accommodate a single pressure-indicating valve 1600 on the outer wall of the pressure channel 1606. In one configuration, the single valve 1600 is a pressure relief valve. The pressure relief valve is activated at a set pressure. The pressure of the surgical cavity is indicated based on when the valve is activated. The pressure at which the pressure relief valve 1600 opens can be adjustable. The pressure inside the surgical cavity can therefore be set by varying the pressure relief valve 1600 setting, allowing the gases supply to provide a continuous flow of gases at a substantially stable pressure. When the pressure inside the surgical cavity exceeds the pressure relief valve 1600 set pressure, the pressure relief valve 1600 can open to vent out the excess pressure from the system and maintain a desired pressure cap in the surgical cavity. The gases supply can be configured to supply gases at a pressure higher than the set pressure to reduce the likelihood of the pressure inside the surgical cavity falling below the set pressure.

Accordingly, the valves in Figures 16A and 16B can be implemented in two modes. In a first mode, a single valve may be activated at different preset pressures, such as shown in Figure 16B. In a second mode, such as shown in Figure 16A, a plurality of valves can be activated as the pressure in the channel 1606 increases. For example, at a first low pressure, the first valve is activated. At a second pressure higher than the first pressure, the first two or three valves are activated. At a pressure higher than the second pressure, all valves may be activated.

The pressure-indicating valve(s) 1600 may be powered by power lines that are routed through the gases delivery conduit 1612. The conduit 1612 may also include the sensor described above. The valve(s) 1600 may also be configured for wireless communication with the processor.

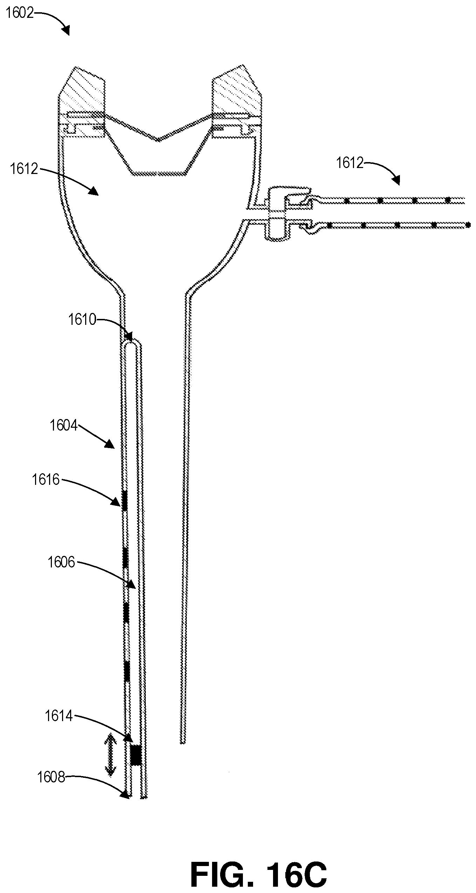

Figures 16C-16E illustrate schematically cross-sectional views of a cannula 1602 (which can be a gases delivery cannula) incorporating an example pressure-indicating slider 1614. As shown in Figures 16C-16E, a pressure channel 1606 is located within the cannula shaft 1604 of the cannula 1602. The outer wall of the cannula shaft 1604 and the outer wall of the pressure channel 1606 can overlap at least partially. The pressure channel 1606 can have an open end 1608 at a distal end of the cannula shaft 1604 configured to be inserted into and in fluidic communication with the surgical cavity. The pressure channel 1606 can have a closed end 1610 opposite the open end. The closed end 1610 can be located nearer to the cannula body 1612 than the open end 1608. The slider can translate linearly along the channel 1606. When the pressure inside the surgical cavity increases, the increased pressure can cause the slider 1614 to move (for example, proportional to the increase in the pressure) closer to the closed end 1610. When the pressure inside the surgical cavity decreases, the decreased pressure can cause the slider 1614 to move (for example, proportional to the decrease in the pressure) closer to the open end 1608.

The change in position of the slider 1614 inside the channel 1606 can be determined using a variety of ways. For example, as shown in Figure 16C, the channel 1606 can include a plurality (for example, three, four, or more) of switches 1616 along at least a portion of a length of the channel 1606. The switches 1616 can include sensors configured to detect when at least a portion of the slider 1614 is level or substantially level with one of the switches 1616. The sensor can be a mechanical sensor, for example, limit switches, electrical contacts, switch buttons, or otherwise. The sensor can also be contactless, for example, a magnetic sensor, capacitive sensor, or otherwise. The plurality of switches 1616 can be located along the length of the channel 1606 so that each switch 1616 corresponds to a different pressure value. The switches 1616 closer to the closed end 1610 correspond to a higher pressure value than the switches 1616 closer to the open end 1608.

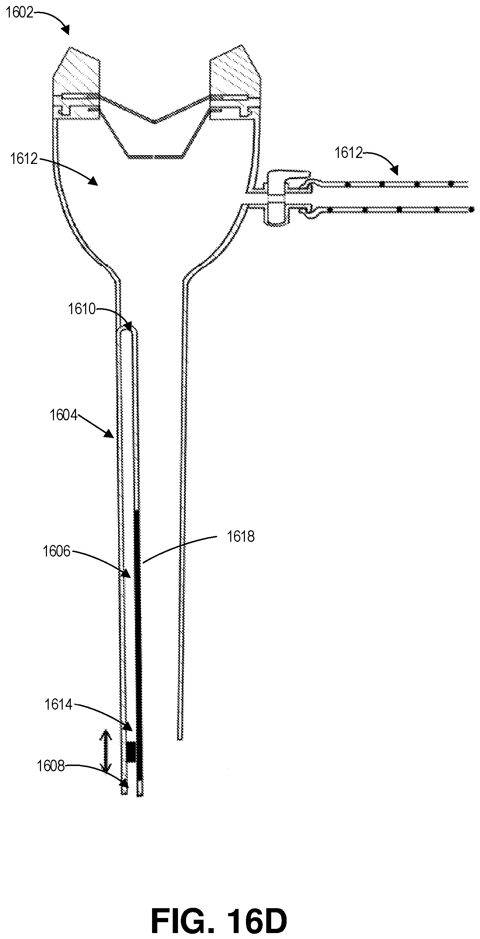

As another example, such as shown in Figure 16D, the channel 1606 can include a linear position sensor 1618 along at least a portion of a length of the channel 1606. The linear position sensor 1618 can detect a position of the slider 1614. In a configuration, the linear position sensor 1618 can provide continuous readings of the position of the slider 1614 to the processor, the readings corresponding to the pressure inside the surgical cavity. The linear position sensor can include any type of sensor configured to detect a linear position, for example but not limited to a linear encoder or a linear potentiometer.

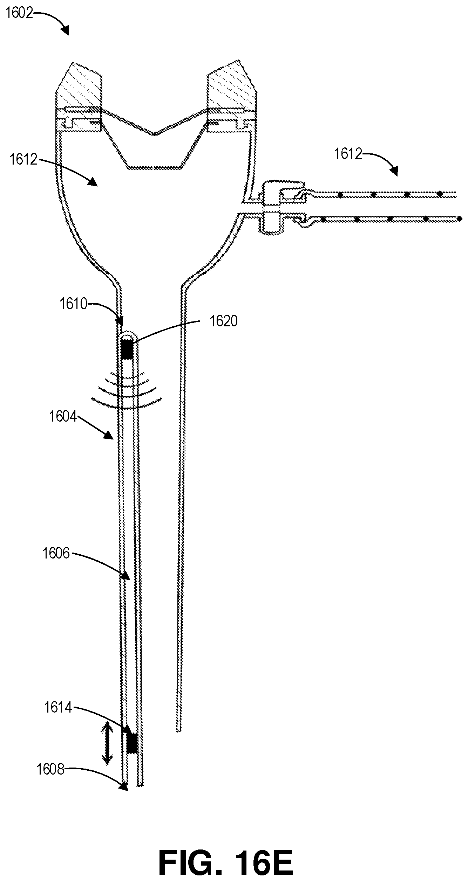

In another example, such as shown in Figure 16E, the channel 1606 can include a non-contact distance sensor 1620. The sensor 1620 can send a signal toward the slider 1614 and the processor can calculate a distance between the sensor 1620 and the slider 1614 based at least in part on the signal from the sensor 1620. The distance between the sensor 1620 and the slider 1614 can correspond to (for example, be proportional to) the pressure inside the surgical cavity. The sensor can include a signal emitter and a signal receiver. The signal emitter can be located at one end of the channel 1620, such as being located close to the closed end 1610 as shown in Figure 16E. The receiver can be located on a surface of the slider 1614 facing the sensor 1620. Alternatively, the receiver can be located at or near the same location as the emitter and the surface of the slider 1614 facing the sensor 1620 can include a reflector. The signal can be wave-based (for example, electromagnetic waves, acoustic waves, or otherwise), field-based (for example, magnetic field, electrostatic field, or otherwise), or any other non-contact distance-sensing signal.

The switches 1616, the position sensor 1618, and/or the non-contact distance sensor 1620 may be powered by power lines that are routed through the gases delivery conduit 1612. The switches 1616, the position sensor 1618, and/or non-contact distance sensor 1620 may also be configured for wireless communication with the processor.

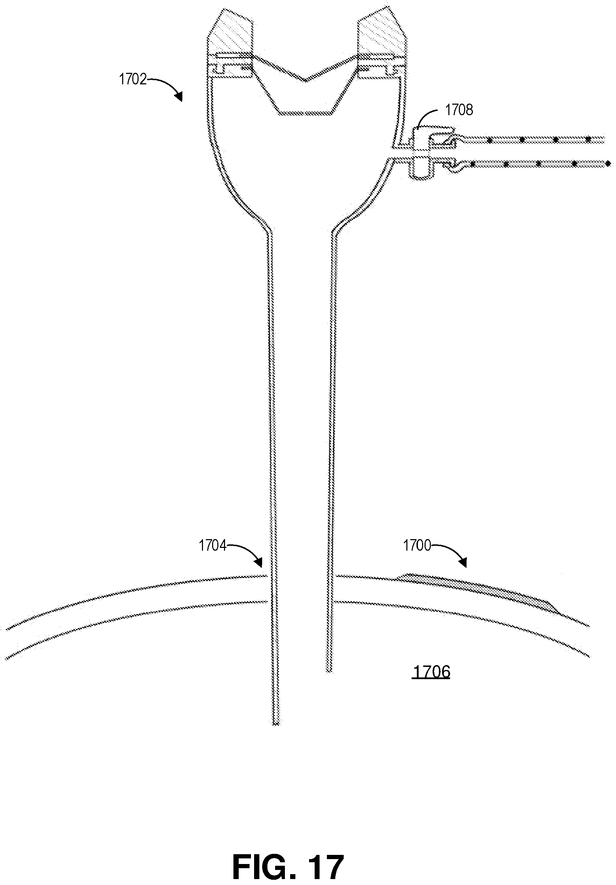

As shown in Figure 17, the cannula 1702 can be inserted into the surgical cavity 1706 at an incision site 1704. A sensing apparatus 1700 can be secured to the patient's skin adjacent or near the incision site 1704, such as via adhesives or otherwise. The sensing apparatus 1700 can be a strain sensing apparatus, for example, a strain gauge. Pressure inside the surgical cavity 1706 can cause the skin to which the sensing apparatus 1700 is secured to expand or contract. The strain sensing apparatus 1700 may include a plurality of strain sensors that are configured to sense the movement, that is, strain of the patient's skin. The movement corresponds to the pressure in the surgical cavity.

The strain sensing apparatus 1700 is closely attached to the skin such that the strain sensing apparatus 1700 can sense the skin stretching or relaxing due changes in the pressure within the surgical cavity. The strain sensing arrangement 1700 may include a mounting device that includes a first element that is attached to the skin of the patient and a second element that is attached to the strain sensing apparatus 1700. The first and second element can be removably attached to each other. In one example, the first and second elements are removably attached by corresponding portions of hook and loop fasteners. The first element can include a patient contacting side that includes a hydrocolloid gel configured to adhere removably to the patient's skin. The second element can include a carrier layer that supports the hook and loop fasteners. The carrier layer may be silicon, a hydrocolloid gel, or another suitable material.

The deformation under pressure of the surface of the skin to which the sensing apparatus 1700 is secured can be used, for example, via calibration, to estimate the internal pressure of the surgical cavity.

Calibration of the sensing apparatus 1700 can be performed, for example, by using pressure measurements from other types of pressure sensing of the gases supply and/or surgical cavity while monitoring the strain gauge in the sensing apparatus 1700. The mathematical relationship between the pressure in the surgical cavity and the strain sensed by the strain sensing apparatus 1700 in the form of a strain gauge can be determined experimentally. In one example implementation, the strain gauge is calibrated at a zero position for a predetermine pressure. The predetermined pressure may be an operative pressure or operative pressure range for laparoscopic surgery. If the strain gauge measures an increasing strain, this can correspond to an increase in pressure. A larger negative strain from the zero position may denote a reduction of the pressure in the cavity. The processor may store the mathematical relationship between a detected strain value and a pressure value in the surgical cavity. The processor can use this relationship to provide a pressure reading based on the detected strain. Other calibration methods can also be implemented.

The strain gauge information can be sent back to the processor via wireless transmission (using any wireless communication protocol, such as Wi-Fi, Bluetooth, NFC, etc.) and/or a wired connection. The wired connection can be to the end of the cannula 1702 or to the end of the tube set, for example, the connector 1708. Wires for powering the sensing apparatus 1700 and transporting the sensing signals (for example, the strain gauge signals) may extend from the end of the cannula 1702 or may extend from the connector 1708 of the tube-set.

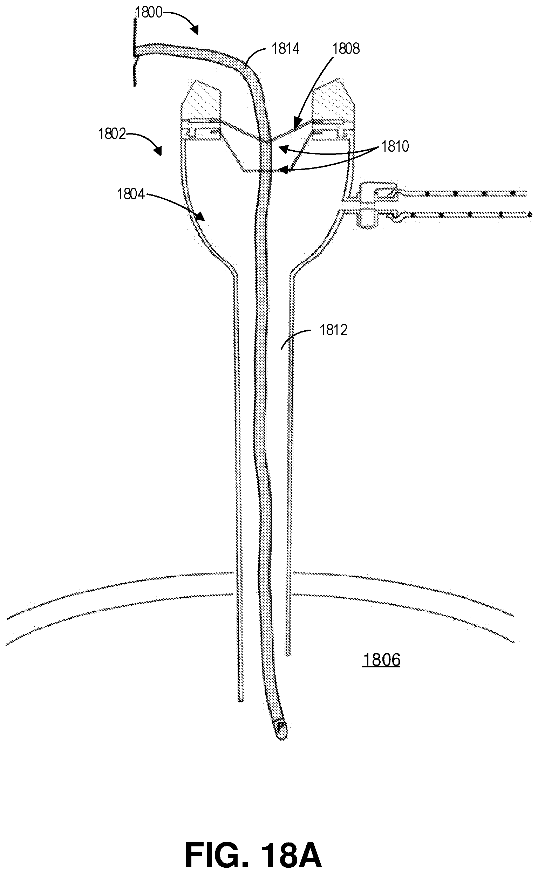

Figure 18A illustrates an example pressure-sensing probe 1800 inserted into the surgical cavity 1806 through a cannula 1802 (which can be a standard cannula, or other cannulas as disclosed elsewhere herein). The pressure-sensing probe can include a flexible and/or malleable probe terminating at a pressure sensor. The probe can be elongate. The seals 1808 of the cannula body 1804 (which provide a seal against an instrument inserted into the cannula and provides a gases seal) can include an orifice 1810 allowing the pressure-sensing probe 1800 to be extended therethrough, past the cannula body 1804 and shaft 1812 into the surgical cavity 1806. The pressure-sensing probe 1800 can terminate anywhere inside the surgical cavity 1806 or inside the shaft 1812 to measure the pressure inside the surgical cavity directly. The pressure-sensing probe 1800 can have an elongated body thin enough to allow a medical instrument (such as a laparoscope, a surgical tool, or otherwise) to pass through the lumen of the cannula shaft 1812. The pressure-sensing probe may be inserted into a cannula that also receives an instrument through it. Alternatively, the pressure-sensing probe may be inserted through a venting cannula or venting port that is used to vent gases from the surgical cavity. Alternatively, the pressure-sensing probe may be inserted through a further separate cannula that is used for sensing purposes. The elongate body of the pressure-sensing probe 1800 can be flexible. The elongate body of the pressure-sensing probe 1800 may be malleable. For example, the elongate body can include a malleable wire extending through the body. The wire can be bent 1814 into a desired position to locate the pressure- sensing probe in a desired location and to maintain its place relative to the cannula 1802 and the surgical cavity 1806. The pressure-sensing probe 1800 can be electrically connected to the gases supply. The pressure-sensing probe 1800 is electrically coupled to the processor that is used to process signals from the pressure-sensing probe 1800 to determine a pressure based on the detected signals. The pressure-sensing probe may also be wirelessly connected to the processor.

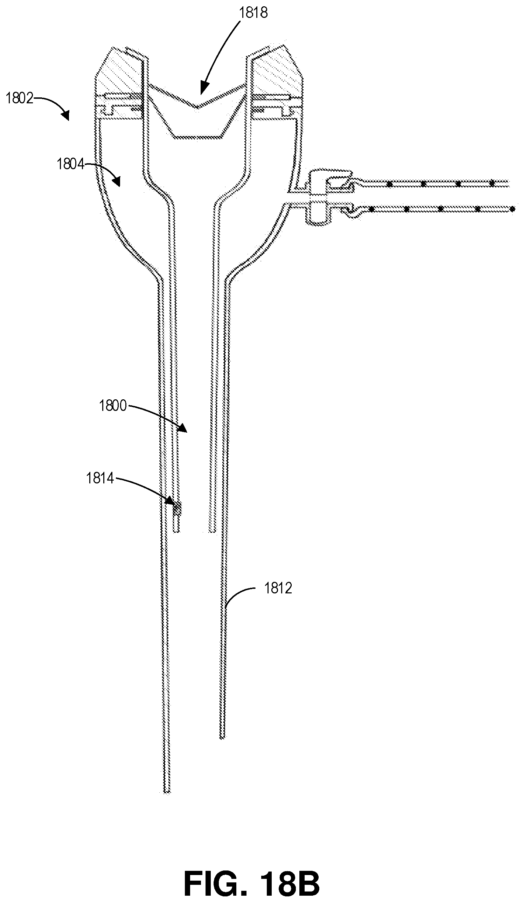

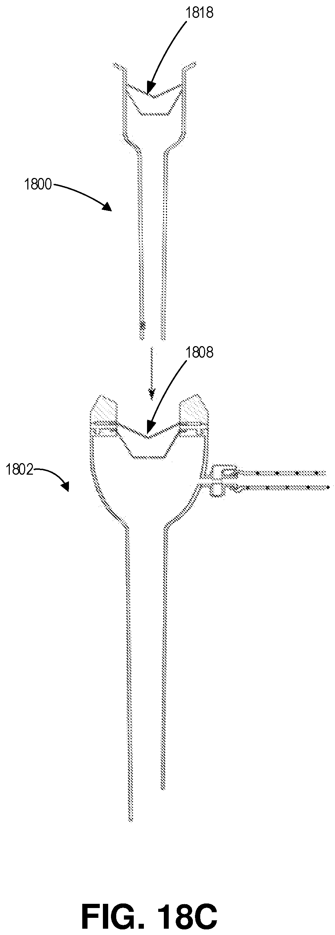

Figure 18B and 18C illustrate a pressure-sensing probe 1800 that can be inserted into the cannula 1802 (which can be a standard cannula) past the seals 1808 of the cannula 1802. The pressure-sensing probe 1800 can have any features of the probe 1800 in Figure 18A. The seals 1808 provide a seal against an instrument inserted into the cannula 1802 and provide a gases seal. The pressure-sensing probe 1800 can include one or more seals 1818 to provide a seal with the instrument. The probe 1800 provides an instrument passage as well as an extension to allow positioning of the pressure sensor into the cannula and surgical cavity. Seals 1818 can be located on the pressure-sensing probe 1800 such that when the pressure-sensing probe 1800 is inserted as shown in Figure 18B, the pressure-sensing probe 1800 can act as an internal cannula and the seals 1818 can form a seal at an opening of the cannula body 1804. The pressure-sensing probe 1800 can terminate along a length of the cannula shaft 1812. A pressure sensor 1814 can be located near the end of the pressure-sensing probe 1800 inside the lumen of the cannula shaft 1812. The pressure inside the lumen of the cannula shaft 1812, which is expected to be substantially the same as the pressure inside the surgical cavity, can be measured by the pressure sensor 1814. The signals relating to the pressure values can be transmitted to the processor via wireless transmission and/or a wired connection as described above for processing and determining a pressure value. The processor may be located at the gases supply and/or within the humidifier, or the processor may be a remote processor.

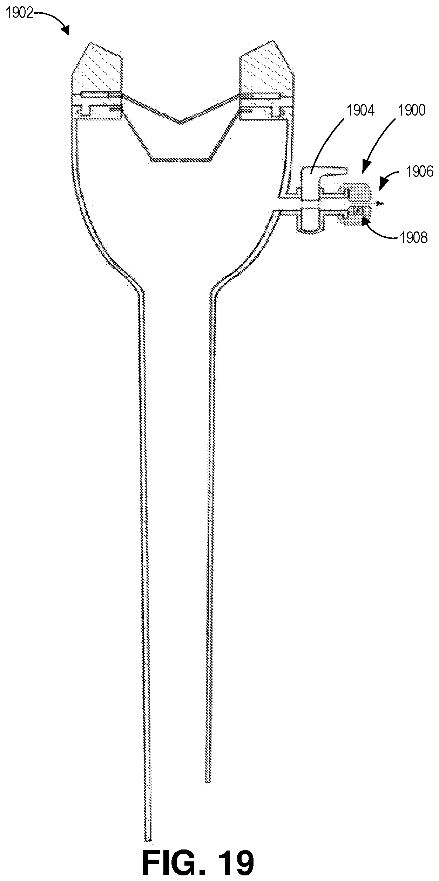

As shown in Figure 19, a pressure-sensing attachment 1900 can be coupled to the gases port 1904 of a venting cannula 1902 (which can be the venting cannula 22 in Figure 2A). As similarly shown in Figure 2A, the gases port 1904 of the venting cannula 1902 can remain open during use of the medical gases delivery system, such as in a surgery. The pressure-sensing attachment 1900 can include a known orifice (similar to the orifice 500 in Figure 5) with a leak. The pressure-sensing attachment 1900 can also include a pressure and/or flow sensor 1906 configured to measure the flow rate of the leak. The measured leak is correlated to the pressure being provided as the relationship between the pressure and the leak flow rate is known. The flow rate measurements can be used to estimate the pressure inside the surgical cavity. The flow sensor can also be configured to measure the pressure inside the surgical cavity. A venting cannula or venting port is normally used during surgeries. The sensing attachment 1900 can be attached to the venting cannula. Alternatively, the sensing attachment 1900 can couple to another venting device, for example, a smoke evacuation system or a smoke filter attachment. A pressure sensing attachment can also be coupled to the gases port of a different cannula other than the venting cannula 1902.

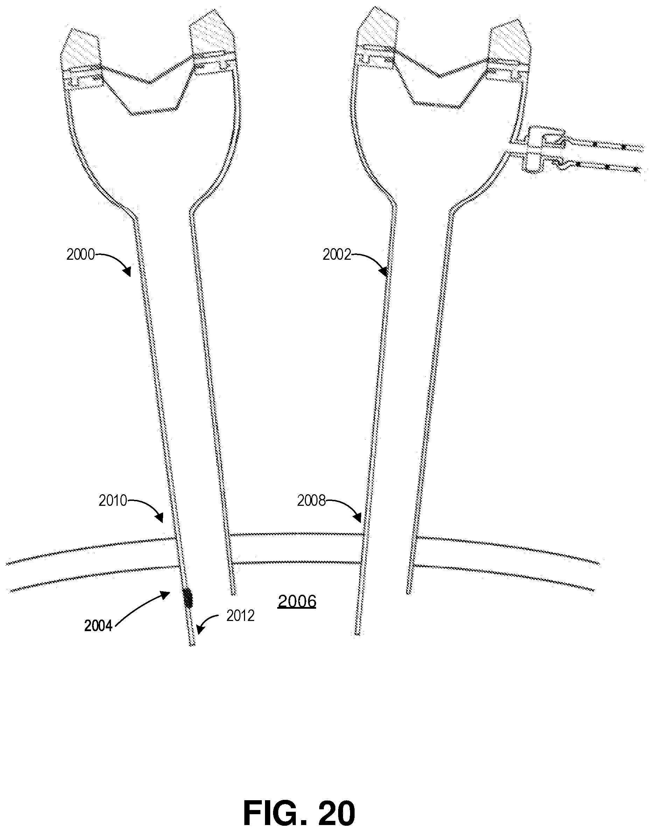

A pressure-sensing cannula 2000, such as shown in Figure 20, can be used to measure pressure inside a surgical cavity 2006. The discrete gases delivery cannula 2002, which is a separate gases delivery cannula, can be inserted into the surgical cavity 2006 via a first incision site 2008 on the patient's skin. The pressure-sensing cannula 2000 can be inserted into the surgical cavity 2006 via a second incision site 2010. A pressure sensor 2004, or any other sensor from which pressure can be directly or indirectly calculated, can be located near the free end of the shaft 2012 of the pressure-sensing cannula 2000. The pressure sensor 2004 can be located within (or outside) the surgical cavity 2006 when the pressure-sensing cannula 2000 is inserted into the surgical cavity 2006. The sensor measurements can be sent to the gases supply via wireless transmission as described above, or a wired connection, for example, with an electrical signal line that connects the pressure sensor 2004 to the gases supply. The pressure-sensing cannula 2000 can also incorporate any of the sensing devices and methods described herein.

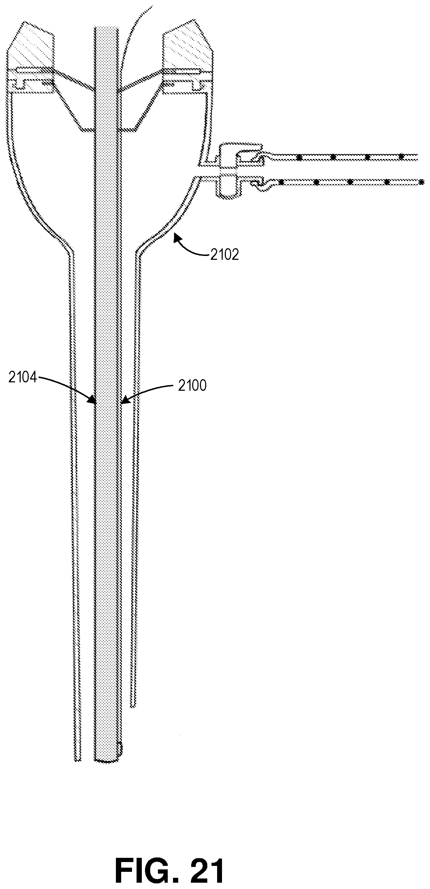

Figure 21 illustrates a pressure sensor attachment 2100 that can be releasably attached to (such as wrapped around or adhered to) a medical instrument (for example, a scope) 2104. When the medical instrument is inserted into a gases delivery cannula 2102, the pressure sensor attachment 2100 can measure pressure inside the surgical cavity. The pressure measurements can be sent to the gases supply via wired or wireless transmission as described above.

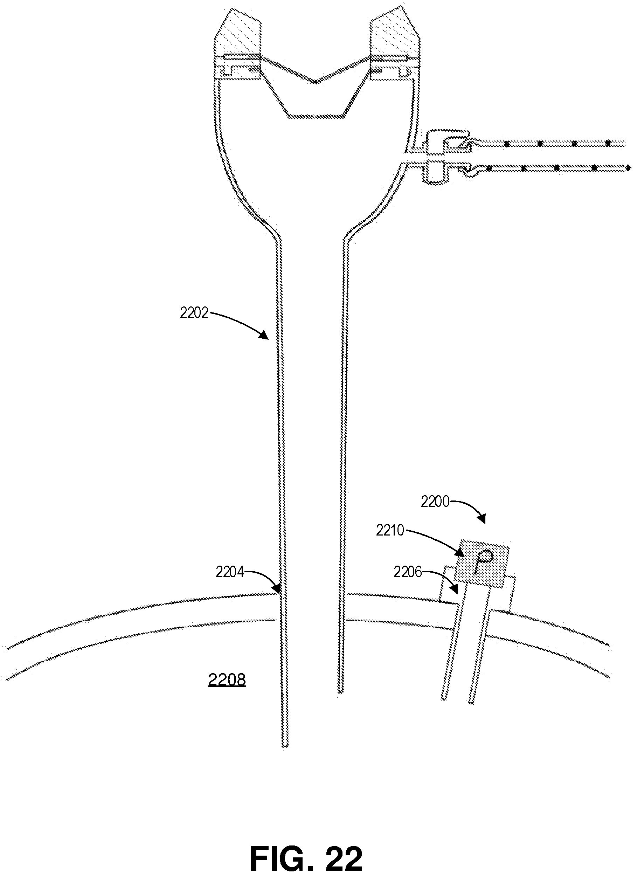

Figure 22 illustrates a cross-sectional view of a cannula 2202 (which may be a gases delivery cannula) inserted into a surgical site 2208 and a separate pressure port 2200. The cannula 2202 can be inserted into the surgical cavity 2208 via a first incision site 2204 on the patient's skin. The pressure port 2200 can be inserted into the surgical cavity 2208 via a second incision site 2206 near the first incision site 2204. The pressure port 2200 can be inserted into the surgical cavity 2208 during the same step in the surgical procedure. The pressure port 2200 can include a pressure sensor 2210 configured to measure directly pressure inside the surgical cavity 2208. The sensor measurements can be sent to the gases supply via wireless transmission as described above, or a wired connection. The pressure port 2200 can also function in the same way as the known orifice in Figure 19. The pressure port 2200 may be a leak device with a known orifice and a leak rate that can be measured by a flow sensor. The pressure inside the surgical cavity can be determined based on the measured leak rate as described above.

### Terminology

Examples of medical gases delivery systems and associated components and methods have been described with reference to the figures. The figures show various systems and modules and connections between them. The various modules and systems can be combined in various configurations and connections between the various modules and systems can represent physical or logical links. The representations in the figures have been presented to clearly illustrate the principles and details regarding divisions of modules or systems have been provided for ease of description rather than attempting to delineate separate physical embodiments. The examples and figures are intended to illustrate and not to limit the scope of the disclosure described herein. For example, the principles herein may be applied to a surgical humidifier as well as other types of humidification systems, including respiratory humidifiers.

As used herein, the term "processor" refers broadly to any suitable device, logical block, module, circuit, or combination of elements for executing instructions. For example, the controller 8 can include any conventional general purpose single- or multi-chip microprocessor such as a Pentium^{®} processor, a MIPS^{®} processor, a Power PC^{®} processor, AMD^{®} processor, ARM^{®} processor, or an ALPHA^{®} processor. In addition, the controller 122 can include any conventional special purpose microprocessor such as a digital signal processor or a microcontroller. The various illustrative logical blocks, modules, and circuits described in connection with the embodiments disclosed herein can be implemented or performed with a general purpose processor, a digital signal processor (DSP), an application specific integrated circuit (ASIC), a field programmable gate array (FPGA), or other programmable logic device, discrete gate or transistor logic, discrete hardware components, or any combination thereof designed to perform the functions described herein, or can be a pure software in the main processor. For example, logic module can be a software-implemented function block which does not utilize any additional and/or specialized hardware elements. Controller can be implemented as a combination of computing devices, e.g., a combination of a DSP and a microprocessor, a combination of a microcontroller and a microprocessor, a plurality of microprocessors, one or more microprocessors in conjunction with a DSP core, or any other such configuration.

Data storage can refer to electronic circuitry that allows data to be stored and retrieved by a processor. Data storage can refer to external devices or systems, for example, disk drives or solid state drives. Data storage can also refer to fast semiconductor storage (chips), for example, Random Access Memory (RAM) or various forms of Read Only Memory (ROM), which are directly connected to the communication bus or the controller. Other types of data storage include bubble memory and core memory. Data storage can be physical hardware configured to store data in a non-transitory medium.

Although certain embodiments and examples are disclosed herein, inventive subject matter extends beyond the specifically disclosed embodiments to other alternative embodiments and/or uses, and to modifications and equivalents thereof. Thus, the scope of the disclosure or embodiments appended hereto is not limited by any of the particular embodiments described herein. For example, in any method or process disclosed herein, the acts or operations of the method or process can be performed in any suitable sequence and are not necessarily limited to any particular disclosed sequence. Various operations can be described as multiple discrete operations in turn, in a manner that can be helpful in understanding certain embodiments; however, the order of description should not be construed to imply that these operations are order dependent. Additionally, the structures described herein can be embodied as integrated components or as separate components. For purposes of comparing various embodiments, certain aspects and advantages of these embodiments are described. Not necessarily all such aspects or advantages are achieved by any particular embodiment. Thus, for example, various embodiments can be carried out in a manner that achieves or optimizes one advantage or group of advantages as taught herein without necessarily achieving other aspects or advantages as can also be taught or suggested herein.

Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements and/or states. Thus, such conditional language is not generally intended to imply that features, elements and/or states are in any way required for one or more embodiments. As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is otherwise understood with the context as used in general to convey that an item, term, etc. may be either X, Y or Z. Thus, such conjunctive language is not generally intended to imply that certain embodiments require at least one of X, at least one of Y and at least one of Z each to be present. As used herein, the words "about" or "approximately" can mean a value is within ±10%, within ±5%, or within ±1% of the stated value.

Methods and processes described herein may be embodied in, and partially or fully automated via, software code modules executed by one or more general and/or special purpose computers. The word "module" refers to logic embodied in hardware and/or firmware, or to a collection of software instructions, possibly having entry and exit points, written in a programming language, such as, for example, C or C++. A software module may be compiled and linked into an executable program, installed in a dynamically linked library, or may be written in an interpreted programming language such as, for example, BASIC, Perl, or Python. It will be appreciated that software modules may be callable from other modules or from themselves, and/or may be invoked in response to detected events or interrupts. Software instructions may be embedded in firmware, such as an erasable programmable read-only memory (EPROM). It will be further appreciated that hardware modules may comprise connected logic units, such as gates and flip-flops, and/or may comprise programmable units, such as programmable gate arrays, application specific integrated circuits, and/or processors. The modules described herein can be implemented as software modules, but also may be represented in hardware and/or firmware. Moreover, although in some embodiments a module may be separately compiled, in other embodiments a module may represent a subset of instructions of a separately compiled program, and may not have an interface available to other logical program units.

In certain embodiments, code modules may be implemented and/or stored in any type of computer-readable medium or other computer storage device. In some systems, data (and/or metadata) input to the system, data generated by the system, and/or data used by the system can be stored in any type of computer data repository, such as a relational database and/or flat file system. Any of the systems, methods, and processes described herein may include an interface configured to permit interaction with users, operators, other systems, components, programs, and so forth.

It should be emphasized that many variations and modifications may be made to the embodiments described herein, the elements of which are to be understood as being among other acceptable examples. Further, nothing in the foregoing disclosure is intended to imply that any particular component, characteristic or process step is necessary or essential. The scope of the present invention is defined by the claims that follow.

## Claims

1. A tube for delivering a flow of gases in a medical gases delivery system that is configured to supply gases to a patient, the tube comprising:
a first end (304) and a second end (306);
• a first connector at the first end and a second connector at the second end of the tube;
• an inner conduit (302) defining a lumen (310) to transport gases therethrough;
• an outer conduit (300), coaxial with and surrounding the inner conduit such that the inner and outer conduits define a dual wall tube defining a secondary lumen (308) between walls of the inner and outer conduits; and
• a pressure sensor (312) arranged in the secondary lumen (308) to measure a pressure in the secondary lumen;
• wherein the pressure sensor (312) is configured to be in electrical communication with a processor; and
wherein the secondary lumen (308) is not configured for delivery or removal of gases therethrough.

2. The tube of Claim 1, wherein the pressure sensor (312) is located at the first connector or at the second connector.

3. The tube of Claim 1, wherein the tube comprises a heater wire (406).

4. The tube of Claim 1, wherein the pressure sensor (312) comprises an expansion ring configured to deform in response to the pressure, and wherein the expansion ring is located at the first connector or at the second connector.

5. The tube of Claim 4, wherein the expansion ring comprises a strain sensor (404) that is in electrical communication with a heater wire (406).

6. The tube of Claim 1, wherein the pressure sensor (312) comprises a strain sensor (404) in the tube, the strain sensor (404) configured to deform in response to the pressure.

7. The tube of Claim 6, wherein the tube is configured such that, in use, gases from a surgical cavity can enter the secondary lumen (308) due to a pressure differential between the surgical cavity and the secondary lumen.

8. The tube of any one of Claims 1 to 7, wherein the processor is embedded within the tube.

9. The tube of any one of Claims 1 to 8, further comprising a filter (6) that is configured to be located downstream of a humidifier.

10. A tube-set comprising: a delivery tube configured to connect a gases supply to a humidifier; and a supply tube comprising the tube of any one of Claims 1 to 9 configured to connect the humidifier to a cannula (15) or to a medical instrument such as a diffuser (220) or a directed gas flow accessory.

11. A surgical humidification system, the system comprising:
the tube of any of Claims 1 to 9; and
a humidifier comprising a humidification chamber (5), the first or second connector coupled to an outlet (11) of the humidification chamber.

12. The system of Claim 11, wherein the pressure sensor (312) is configured to detect over-pressure and/or under-pressure in the system, and/or undesirably high or low flow rates of the flow of gases;
and/or wherein signals from the pressure sensor (312) are configured to be used to detect undesirable or improper connections, and/or inappropriate connections for a particular surgical application.

13. The system of Claim 11 or 12, comprising a processor in electrical communication with the pressure sensor (312) and configured to determine a pressure at a surgical site based on readings from the pressure sensor.

14. The system of Claim 11 or 12, comprising a processor in electrical communication with the pressure sensor (312), wherein the processor is part of a controller (21) of the gases supply or the processor is part of a controller of the humidifier.

## Patentansprüche

1. Schlauch zum Fördern eines Gasstroms in einem medizinischen Gasförderungssystem, das dazu ausgebildet ist, einem Patienten Gase zuzuführen, wobei der Schlauch aufweist:
ein erstes Ende (304) und ein zweites Ende (306);
• einen ersten Konnektor an dem ersten Ende und einen zweiten Konnektor an dem zweiten Ende des Schlauchs;
• eine innere Leitung (302), die ein Lumen (310) definiert, um Gase hindurch zu transportieren;
• eine äußere Leitung (300), die koaxial zu der inneren Leitung verläuft und diese umgibt, sodass die innere und die äußere Leitung einen doppelwandigen Schlauch definieren, der zwischen den Wänden der inneren und der äußeren Leitung ein sekundäres Lumen (308) definiert; und
• einen Drucksensor (312), der in dem sekundären Lumen (308) angeordnet ist, um einen Druck in dem sekundären Lumen zu messen;
• wobei der Drucksensor (312) dazu ausgebildet ist, in elektrischer Kommunikation mit einem Prozessor zu sein; und
wobei das sekundäre Lumen (308) nicht für eine Zufuhr oder Entfernung von Gasen durch dieses hindurch ausgebildet ist.

2. Schlauch nach Anspruch 1, wobei der Drucksensor (312) an dem ersten Konnektor oder an dem zweiten Konnektor angeordnet ist.

3. Schlauch nach Anspruch 1, wobei der Schlauch einen Heizdraht (406) aufweist.

4. Schlauch nach Anspruch 1, wobei der Drucksensor (312) einen Expansionsring aufweist, der dazu ausgebildet ist, sich in Reaktion auf den Druck zu verformen, und wobei der Expansionsring an dem ersten Konnektor oder an dem zweiten Konnektor angeordnet ist.

5. Schlauch nach Anspruch 4, wobei der Expansionsring einen Dehnungssensor (404) aufweist, der in elektrischer Kommunikation mit einem Heizdraht (406) ist.

6. Schlauch nach Anspruch 1, wobei der Drucksensor (312) einen Dehnungssensor (404) in dem Schlauch aufweist, wobei der Dehnungssensor (404) dazu ausgebildet ist, sich in Reaktion auf den Druck zu verformen.

7. Schlauch nach Anspruch 6, wobei der Schlauch so ausgebildet ist, dass, im Betrieb, Gase aus einem chirurgischen Hohlraum aufgrund einer Druckdifferenz zwischen dem chirurgischen Hohlraum und dem sekundären Lumen (308) in das sekundäre Lumen (308) eintreten können.

8. Schlauch nach einem der Ansprüche 1 bis 7, wobei der Prozessor innerhalb des Schlauchs eingebettet ist.

9. Schlauch nach einem der Ansprüche 1 bis 8, ferner aufweisend einen Filter (6), der dazu ausgebildet ist, stromabwärts einer Befeuchtungseinrichtung angeordnet zu werden.

10. Schlauch-Set, aufweisend:
einen Förderschlauch, der dazu ausgebildet ist, eine Gasversorgung mit einer Befeuchtungseinrichtung zu verbinden; und einen Versorgungsschlauch, der den Schlauch nach einem der Ansprüche 1 bis 9 aufweist und dazu ausgebildet ist, die Befeuchtungseinrichtung mit einer Kanüle (15) oder mit einem medizinischen Instrument wie einem Diffusor (220) oder einem gerichteten Gasflusszubehör zu verbinden.

11. Chirurgisches Befeuchtungssystem, wobei das System aufweist:
den Schlauch nach einem der Ansprüche 1 bis 9; und
eine Befeuchtungseinrichtung, die eine Befeuchtungskammer (5) aufweist, wobei der erste oder der zweite Konnektor an einem Auslass (11) der Befeuchtungskammer gekoppelt ist.

12. System nach Anspruch 11, wobei der Drucksensor (312) dazu ausgebildet ist, einen Überdruck und/oder einen Unterdruck in dem System und/oder unerwünscht hohe oder niedrige Durchflussraten des Gasstroms zu detektieren;
und/oder wobei Signale des Drucksensors (312) dazu ausgebildet sind, verwendet zu werden, um unerwünschte oder unsachgemäße Verbindungen und/oder ungeeignete Verbindungen für eine bestimmte chirurgische Anwendung zu detektieren.

13. System nach Anspruch 11 oder 12, das einen Prozessor in elektrischer Kommunikation mit dem Drucksensor (312) aufweist und dazu ausgebildet, einen Druck an einer chirurgischen Stelle basierend auf Messwerten des Drucksensors zu bestimmen.

14. System nach Anspruch 11 oder 12, das einen Prozessor in elektrischer Kommunikation mit dem Drucksensor (312) aufweist, wobei der Prozessor Teil einer Steuerung (21) der Gasversorgung ist oder wobei der Prozessor Teil einer Steuerung der Befeuchtungseinrichtung ist.

## Revendications

1. Tube pour distribuer un flux de gaz dans un système de distribution de gaz médicaux qui est configuré pour fournir des gaz à un patient, le tube comprenant :
une première extrémité (304) et une seconde extrémité (306) ;
• un premier raccord sur la première extrémité et un second raccord sur la seconde extrémité du tube ;
• un conduit interne (302) définissant une lumière (310) pour transporter des gaz à travers celle-ci ;
• un conduit externe (300), coaxial avec et entourant le conduit interne de sorte que les conduits interne et externe définissent un tube à double paroi définissant une lumière secondaire (308) entre les parois des conduits interne et externe ; et
• un capteur de pression (312) agencé dans la lumière secondaire (308) pour mesurer une pression dans la lumière secondaire ;
• dans lequel le capteur de pression (312) est configuré pour être en communication électrique avec un processeur ; et
dans lequel la lumière secondaire (308) n'est pas configurée pour distribuer ou évacuer des gaz à travers celle-ci.

2. Tube selon la revendication 1, dans lequel le capteur de pression (312) est situé au niveau du premier raccord ou au niveau du second raccord.

3. Tube selon la revendication 1, dans lequel le tube comprend un fil chauffant (406).

4. Tube selon la revendication 1, dans lequel le capteur de pression (312) comprend une bague d'expansion configurée pour se déformer en réponse à la pression, et dans lequel la bague d'expansion est située au niveau du premier raccord ou au niveau du second raccord.

5. Tube selon la revendication 4, dans lequel la bague d'expansion comprend un capteur de déformation (404) qui est en communication électrique avec un fil chauffant (406).

6. Tube selon la revendication 1, dans lequel le capteur de pression (312) comprend un capteur de déformation (404) dans le tube, le capteur de déformation (404) étant configuré pour se déformer en réponse à la pression.

7. Tube selon la revendication 6, dans lequel le tube est configuré de sorte que, lors de son utilisation, des gaz provenant d'une cavité chirurgicale puissent pénétrer dans la lumière secondaire (308) en raison d'un différentiel de pression entre la cavité chirurgicale et la lumière secondaire.

8. Tube selon l'une quelconque des revendications 1 à 7, dans lequel le processeur est incorporé dans le tube.

9. Tube selon l'une quelconque des revendications 1 à 8, comprenant en outre un filtre (6) qui est configuré pour être situé en aval d'un humidificateur.

10. Ensemble de tubes comprenant : un tube de distribution configuré pour raccorder une alimentation en gaz à un humidificateur ; et un tube d'alimentation comprenant le tube selon l'une quelconque des revendications 1 à 9 configuré pour raccorder l'humidificateur à une canule (15) ou à un instrument médical tel qu'un diffuseur (220) ou un accessoire à flux de gaz dirigé.

11. Système d'humidification chirurgicale, le système comprenant :
le tube selon l'une quelconque des revendications 1 à 9 ; et
un humidificateur comprenant une chambre d'humidification (5), le premier ou le second raccord étant couplé à une sortie (11) de la chambre d'humidification.

12. Système selon la revendication 11, dans lequel le capteur de pression (312) est configuré pour détecter une surpression et/ou une dépression dans le système, et/ou des débits de gaz excessivement élevés ou faibles du flux de gaz ;
et/ou dans lequel des signaux du capteur de pression (312) sont configurés pour servir à détecter des raccordements inopportuns ou inappropriés, et/ou des raccordements inadéquats pour une application chirurgicale particulière.

13. Système selon la revendication 11 ou 12, comprenant un processeur en communication électrique avec le capteur de pression (312) et configuré pour déterminer une pression au niveau d'un site chirurgical en fonction de mesures du capteur de pression.

14. Système selon la revendication 11 ou 12, comprenant un processeur en communication électrique avec le capteur de pression (312), dans lequel le processeur fait partie d'un dispositif de commande (21) de l'alimentation en gaz ou le processeur fait partie d'un dispositif de commande de l'humidificateur.
